# EUROPÄISCHE PATENTANMELDUNG

(11) **EP 1 440 974 A2**
(43) Veröffentlichungstag der Anmeldung: **28.07.2004**
(21) Anmeldenummer: 04000399.8
(22) Anmeldetag: 10.01.2004
(51) Int. Cl.: C07D 495/04, C08F 28/06, C08G 61/12

(54) **Alkylendioxythiophene und Poly(alkylendioxythiophene) mit mesogenen Gruppen**

(30) Priorität: 21.01.2003 DE 10302086
(71) Anmelder: Bayer Chemicals AG, 51368 Leverkusen (DE)
(72) Erfinder: Reuter, Knud, Dr., 47800 Krefeld (DE); Karbach, Alexander, Dr., 47800 Krefeld (DE); Ritter, Helmut, Prof. Dr., 42111 Wuppertal (DE); Wrubbel, Noelle, 40215 Wuppertal (DE)

(57) **Zusammenfassung**

Die Erfindung betrifft neue 3,4-Alkylendioxythiophene gegebenenfalls über ein Bindeglied substituiert mit mesogenen Gruppen und deren polymere Derivate (Poly-(3,4-alkylendioxythiophene)).

## Beschreibung

Die Erfindung betrifft neue 3,4-Alkylendioxythiophene substituiert mit mesogenen Gruppen und deren polymere Derivate (Poly-(3,4-alkylendioxythiophene)).

π-konjugierte Polymere zeigen wegen der erheblichen Delokalisierung der π-Elektronen entlang der Hauptkette interessante (nichtlineare) optische Eigenschaften. Nach Oxidation oder Reduktion stellen sie gute elektrische Leiter dar und in der neutralen Form besitzen sie ebenso gute halbleitende Eigenschaften. Daher sind sie für die Anwendung auf Gebieten wie z.B. der Datenspeicherung, der optischen Signalverarbeitung, der Unterdrückung elektromagnetischer Störungen (EMI) und der Sonnenenergieumwandlung, sowie in wiederaufladbaren Batterien, lichtemittierenden Dioden, Feldeffekttransistoren, Leiterplatten, Sensoren und antistatischen Materialien von Interesse.

Poly(3,4-alkylendioxythiophene) und deren Derivate, insbesondere Poly(3,4-ethylendioxythiophen) und dessen Derivate sind, aufgrund hoher Leitfähigkeiten insbesondere in der kationischen (oxidierten) Form und guter Verarbeitbarkeit, von besonderem Interesse (EP-A 339 340 und L. Groenendaal, F. Jonas, D. Freitag, H. Pielartzik & J. R. Reynolds, Adv. Mater. **12**, (2000) S. 481 - 494).

Trotz der guten Eigenschaften des Poly(3,4-ethylendioxythiophen)s hinsichtlich Leitfähigkeit und Verarbeitbarkeit besteht Bedarf an weiteren Verbesserungen. Für die Anwendung beispielsweise in der molekularen Elektronik, Solar- oder Halbleitertechnik werden hohe Anforderungen an die leitfähigen oder halbleitenden Materialien gestellt. Insbesondere spielen Ordnungsphänomene eine große Rolle.

Da die Verarbeitung der leitfähigen oder halbleitenden Polymere vorwiegend aus flüssiger Phase, wie z.B. Lösung, Suspension oder Dispersion, erfolgt, kann auf eine Vororientierung zu hoher Ordnung in fester Phase bisher kein Einfluss genommen werden. Es wäre jedoch denkbar, eine solche Vororientierung durch Selbst-Organisation der Monomere bei der Polymerisation zu erreichen, indem strukturelle Voraussetzungen für Nahordnungen geschaffen werden.

In der Literatur wurden bereits einige Versuche beschrieben, π-konjugierte Polymere herzustellen, die mesogene Gruppen, d.h. Gruppen, welche die Bildung mesogener Phasen begünstigen, als Substituenten tragen.

Auch Versuche zur Herstellung von Thiophenen mit mesogenen Substituenten, bzw. deren polymerer Folgeprodukte, wurden bereits in einigen Veröffentlichungen beschrieben. So beschreibt beispielsweise J. Roncali et al. (Adv. Mater. **1994**, 6(2), S. 138-142) die Synthese von 4-Cyan-4'-(8-(3-thienyl)oxy)biphenyl, dessen flüssigkristallines Verhalten sowie die elektrochemische Polymerisation zum entsprechenden Polythiophen. Dessen elektrische Leitfähigkeit wird mit 0,01 bis 0,1 S/cm angegeben. Akagi et al. berichten in Synthetic Metals **1999**, 102, S. 1291, über flüssigkristalline Polythiophene mit chiralen mesogenen Gruppen als Substituenten ohne weitere Informationen zu elektrischen Eigenschaften. Kijima et al. berichtet über ein kationisches, Viologen-substituiertes flüssigkristallines Polythiophen, beschreibt dessen Redoxverhalten und die elektrische Leitfähigkeit von 3,5 x 10⁻³ S/cm im J₂dotierten Zustand, welche jedoch im Hinblick auf oben genannte Anwendungen äußerst niedrig ist (Chem. Letters **2000**, S. 936 - 937). Yagci et al. beschreibt die Herstellung des flüssigkristallinen 3-Thiophenessigsäure-cholesterylesters, der chemisch und elektrochemisch oxidativ zum Polythiophen mit einer Leitfähigkeit von 0,05 S/cm polymerisiert wurde (J. Mater. Sci. **2002**, 37, S. 1767 - 1775).

Für alle in den genannten Literaturstellen beschriebenen Polythiophene sind zwei strukturell bedingte Nachteile charakteristisch. Erstens führt die ausschließlich in Position 3 der Thiophen-Einheit vorliegende Substitution zu Nebenreaktionen bei der Polymerisation, da sich als Substituent in 4-Position ein reaktives H befindet; insbesondere die sogenannte α,β'- Kopplung kann zu strukturellen Defekten, also zur Unterbrechung der Konjugation im Polymeren und verkürzten Konjugationslängen mit der Folge unzulänglicher Leitfähigkeiten führen. Zweitens führen die in den genannten Publikationen ausschließlich in 3-Position C-substituierten Thiophene stets zu nur mäßigen Stabilitäten des hochleitfähigen kationischen Zustandes der Polythiophene (z. B. nach Jod-Dotierung). Solche nachteiligen Effekte sind für analoge, nicht flüssigkristalline Polythiophene gut bekannt, siehe z. B. Leclerc et al., Macromolecules **1991,** 24, S. 455 - 459.

Kumar et al. beschrieb in Synthetic Metals **2001,** 124, S. 471 - 475 ein 3,4-Ethylendioxythiophen mit sperrigen Substituenten sowie dessen elektrochemische Polymerisation zum entsprechend substituierten Poly(3,4-ethylendioxythiophen) sowie Untersuchungen dieser Polymere unter dem Aspekt verbesserter Transparenz. Obwohl der sperrige Substituent als eine potenzielle mesogene Gruppe betrachtet werden kann, wurden keinerlei Hinweise auf flüssigkristallines Verhalten des Monomeren oder Polymeren oder Untersuchungen diesbezüglich gegeben.

Es bestand daher weiterhin Bedarf an 3,4-Alkylendioxythiophenen, die mesogene Gruppen als Substituenten tragen.

Gegenstand der vorliegenden Erfindung sind somit 3,4-Alkylendioxythiophene, dadurch gekennzeichnet, dass sie gegebenenfalls über ein Bindeglied B mit einer mesogenen Gruppe M substituiert sind,
ausgenommen das 3,4-Alkylendioxythiophen der Formel (i)

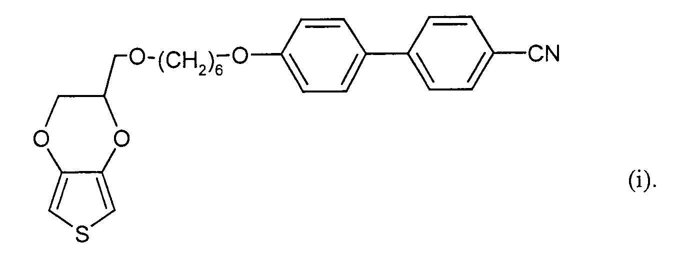

Der Begriff der mesogenen Gruppe ist dem Fachmann bekannt. Darunter werden alle Gruppierungen oder Substanzen verstanden, die geeignet sind, in einem bestimmten Temperaturbereich Mesophasen zu bilden, d.h. Phasen mit einem höheren Ordnungszustand als in isotropen Flüssigkeiten, jedoch mit einem niedrigeren als in Kristallen. Solche Phasen zeigen (noch) die optische Anisotropie von Kristallen und (schon) die Beweglichkeit von durchweg isotropen Flüssigkeiten. Mesophasen werden auch als flüssigkristalline Phasen oder anisotrope Flüssigkeiten bezeichnet.

Die mesogene Gruppe kann im Rahmen der Erfindung eine solche sein, die zur Bildung smektischer, nematischer oder cholesterischer flüssigkristalliner Phasen geeignet ist. Dies können somit stäbchenförmige, scheibenförmige (diskotische) oder cholesterische mesogene Gruppen sein.

Bevorzugt Gegenstand der vorliegenden Erfindung sind 3,4-Alkylendioxythiophene der allgemeinen Formel (I), 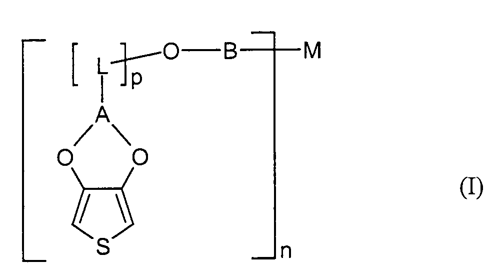 worin
- A: für einen C₁-C₅-Alkylenrest steht, der an beliebiger Stelle durch einen Linker L substituiert ist und gegebenenfalls weitere Substituenten trägt,
- L: für eine Methylengruppe steht,
- p: für 0 oder eine ganze Zahl von 1 bis 6, bevorzugt für 0 oder 1 steht,
- M: für eine n-funktionelle mesogene Gruppe steht,
- n: für eine ganze Zahl von 1 bis 8 steht und
- B: für ein Bindeglied der allgemeinen Formel (B) steht 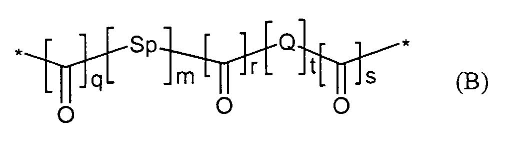 worin
q für 0 oder 1 steht,
r, s für 0 oder 1 stehen, mit der Maßgabe, dass wenn r für 1 steht s für 0 steht und umgekehrt oder beide für 0 stehen können,
t für 0 oder 1 steht,
Sp für einen Spacer ausgewählt aus gegebenenfalls substituierten linearen oder cylischen C₁-C₂₀-Alkylen-, C₅-C₂₀-Arylen-, C₂-C₂₀-Heteroarylen-, wobei zusätzlich noch ein bis drei Heteroatome ausgewählt aus N, O oder S im heteroaromatischen Ring- oder Ringsystem vorhanden sein können, C₆-C₂₀-Aralkylen-, C₂-C₂₀₀-, bevorzugt C₂-C₈₀-Oligo- oder -Polyethergruppen steht,
m für 0 oder 1 steht,
Q für O, S oder NH steht.

Dabei sind sowohl reine 3,4-Alkylendioxythiophene der allgemeinen Formel (I) als auch beliebige Mischungen aus diesen von der Erfindung umfasst.

Besonders bevorzugt sind dies 3,4-Alkylendioxythiophene oder Mischungen aus 3,4-Alkylendioxythiophenen, die eine Struktur der allgemeinen Formel (I-a) und/oder (I-b) besitzen, 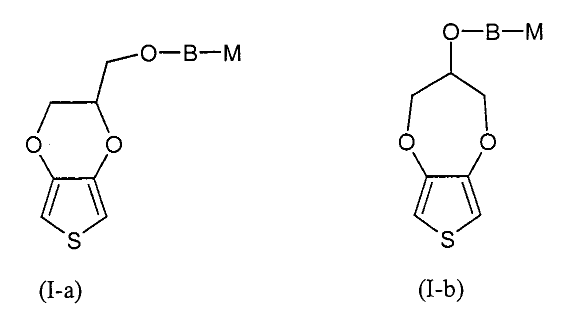 worin

B und M die oben für die allgemeine Formel (I) und/oder die im Folgenden genannte Bedeutung haben.

Sofern es sich um Mischungen aus 3,4-Alkylendioxythiophenen der allgemeinen Formel (I-a) und (I-b) handelt, können im Rahmen der Erfindung 3,4-Alkylendioxythiophene der allgemeinen Formel (I-a) und (I-b) in einem beliebigen Stoffmengenverhältnis in diesem Gemisch enthalten sein. Bevorzugt sind 3,4-Alkylendioxythiophene der allgemeinen Formel (I-a) mit einem Anteil von 65 bis 99,9 %, besonders bevorzugt mit einem Anteil von 75 bis 99,5 %, bezogen auf die Gesamtstoffmenge der Thiophene, enthalten, und die 3,4-Alkylendioxythiophenen der allgemeinen Formel (I-b) sind bevorzugt mit einem Anteil von 0,1 bis 35 %, besonders bevorzugt mit einem Anteil von 0,5 bis 25 %, bezogen auf die Gesamtstoffmenge der Thiophene enthalten, mit der Maßgabe, dass die Summe beider Anteile 100 % ergibt.

### Bevorzugt kann im Rahmen der Erfindung

- M: für eine n-funktionelle Gruppe der allgemeinen Formeln (II-a) oder (II-b) stehen, 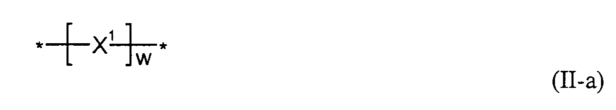 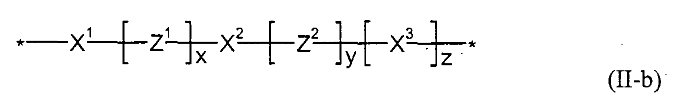 worin
- X¹, X², X³: unabhängig voneinander für gegebenenfalls substituierte Strukturen ausgewählt aus 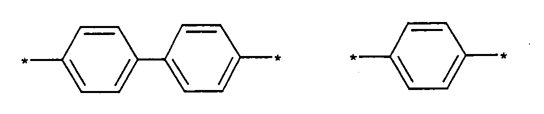 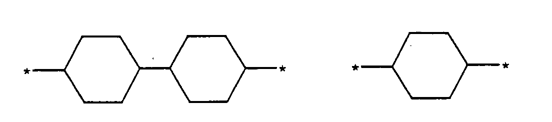 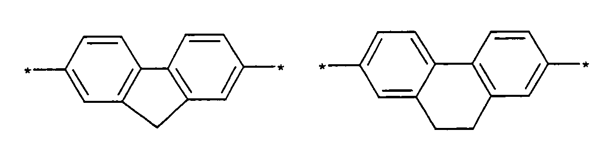 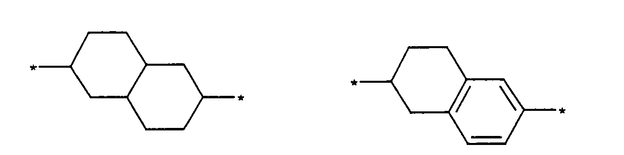 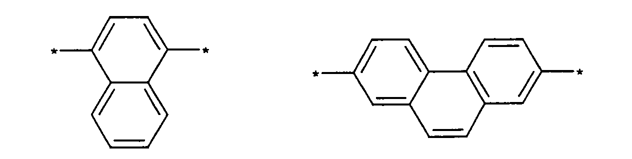 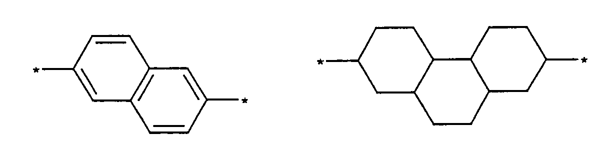 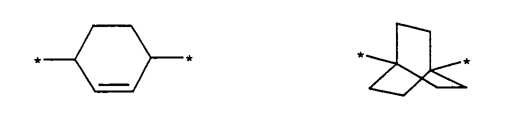 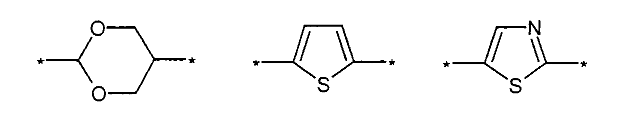 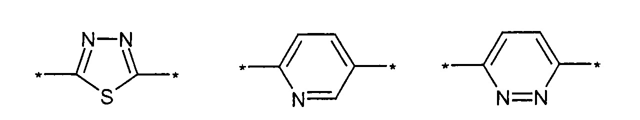 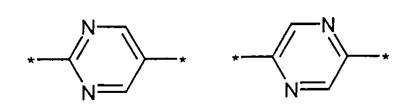 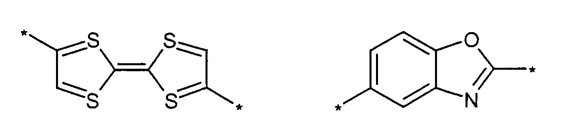 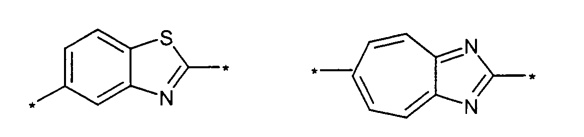 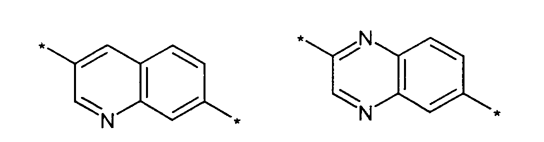 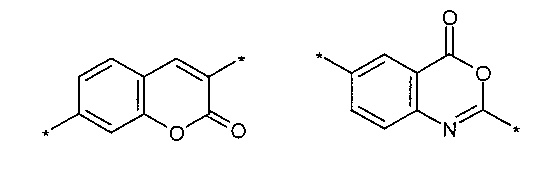 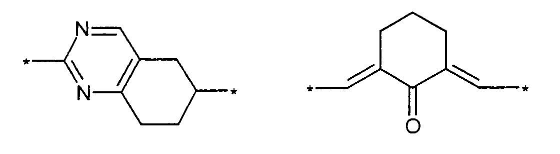 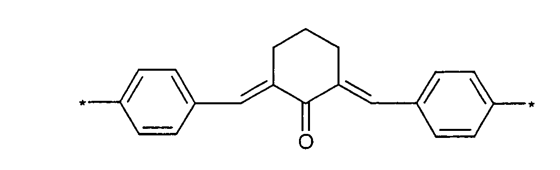
Z¹, Z² unabhängig voneinander für Strukturen ausgewählt aus 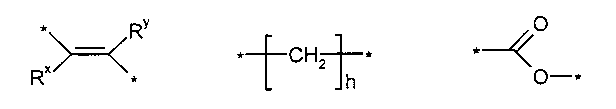 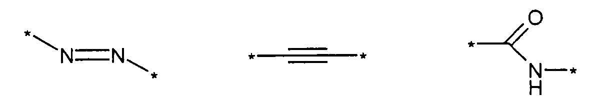 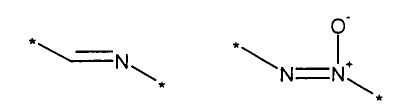 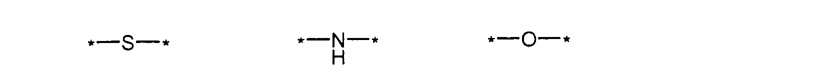 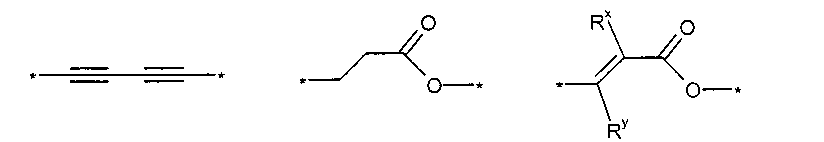 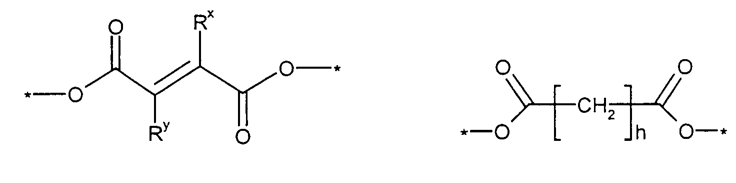 steht,
wobei
- R^{x} und R^{y}: unabhängig voneinander für H, gegebenenfalls substituiertes C₁-C₂₂-Alkyl, C₁-C₂₂-Halogenalkyl, C₁-C₂₂-Alkenyl, C₁-C₂₂-Alkoxy, C₁-C₂₂-Thioalkyl, C₁-C₂₂-Iminoalkyl, C₁-C₂₂-Alkoxycarbonyl, C₁- C₂₂-Alkoxycarbonyloxy, einen Rest einer aliphatischen C₁-C₂₂-Alkancarbonsäure oder der Acrylsäure, Halogen, Pseudohalogen, NO₂, eine Carboxyl- oder eine Hydroxygruppe steht,
- h: für eine ganze Zahl von 1 bis 10 steht,
- w: eine ganze Zahl von 1 bis 5, bevorzugt 1 bis 3, ist,
- x, y, z: unabhängig voneinander für 0 oder 1 stehen, und
- n: für 1 oder 2 steht, wobei
für den Fall, dass n für 1 steht, die Gruppe der allgemeinen Formeln (II-a) oder (II-b) an einer der mit * gekennzeichneten Verknüpfungsstellen eine Flügelgruppe F trägt,
wobei
- F: für H, gegebenenfalls substituiertes C₁-C₂₂-Alkyl, C₁-C₂₂-Halogenalkyl, C₁-C₂₂₋Alkenyl, C₁₋C₂₂-Alkoxy, C₁₋C₂₂₋Thioalkyl, C₁₋C₂₂-Iminoalkyl, C₁-C₂₂-Alkoxycarbonyl, C₁-C₂₂-Alkoxycarbonyloxy, einen Rest einer aliphatischen C₁-C₂₂-Alkancarbonsäure oder der Acrylsäure, Halogen, Pseudohalogen, eine Nitro- (NO₂-), eine Carboxyl-, eine Sulfonsäure- bzw. Sulfonat- oder eine Hydroxygruppe steht.

In bevorzugten Ausführungsformen der Erfindung, worin M für eine n-funktionelle Gruppe der allgemeinen Formel (II-a) steht, steht w für eine ganze Zahl von 2 bis 5, wobei X¹, n und F die oben genannte Bedeutung haben können.

Ein Besonderheit tritt auf, wenn in der allgemeinen Formel (II-a) w für 1 steht. In diesem Fall kann X¹ an einer der mit * gekennzeichneten Verknüpfungsstellen als Flügelgruppe F beispielsweise eine Carboxylgruppe -COOH oder einen Acrylsäurerest, z. B. -CH=CH-COOH, tragen, damit durch Dimerisierung eine mesogene Gruppe gebildet wird.

Daher steht in anderen bevorzugten Ausführungsformen der Erfindung, worin M für eine n-funktionelle Gruppe der allgemeinen Formel (II-a) steht, w für 1, n für 1 und F für eine Carboxylgruppe -COOH oder einen Acrylsäurerest -CH=CH-COOH, insbesondere dann, wenn X¹ für monocyclische Strukturen aus oben für X¹ genannter Auswahl steht.

Als gegebenenfalls vorhandene Substituenten für X¹, X² und X³ kommen beispielsweise lineare, verzweigte oder cyclische C₁-C₂₂-Alkyl, C₁-C₂₂ Halogenalkyl, C₁-C₂₂-Alkenyl, C₁-C₂₂-Alkoxy, C₁-C₂₂-Thioalkyl, C₁-C₂₂-Iminoalkyl, C₁-C₂₂-Alkoxycarbonyl, C₁-C₂₂-Alkoxycarbonyloxy, einen Rest einer aliphatischen C₁-C₂₂-Alkancarbonsäure oder der Acrylsäure, Halogen, Pseudohalogen, NO₂ eine Carboxyl- oder eine Hydroxygruppe steht.

In bevorzugten Ausführungsformen der vorliegenden Erfindung sind X¹, X² und X³ unsubstituiert.

### Weiterhin bevorzugt kann im Rahmen der Erfindung

- M: für eine n-funktionelle Gruppe ausgewählt aus den allgemeinen Formeln (II-c-1) bis (II-c-6) stehen, 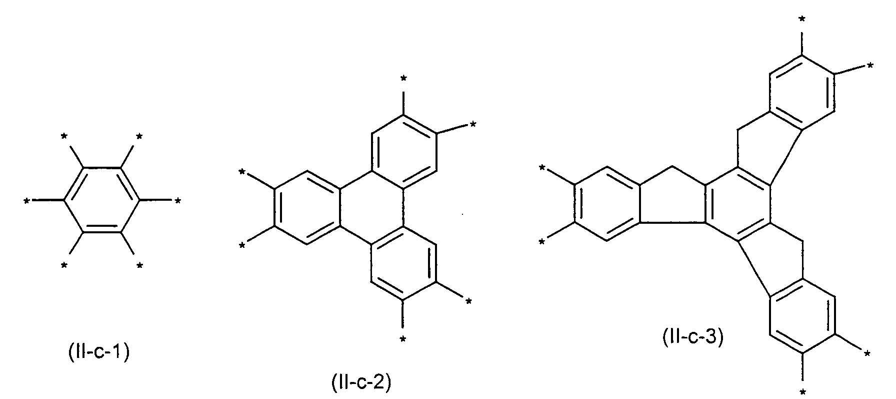 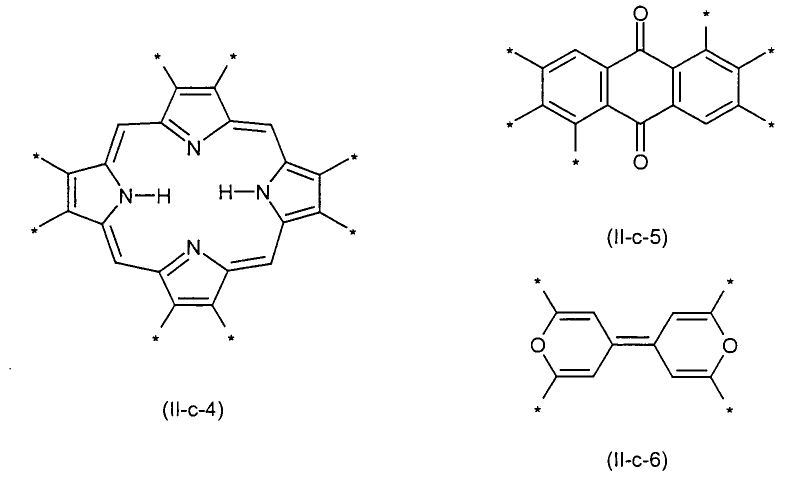 worin
- n: für eine ganze Zahl von 1 bis 8 steht, wobei
für den Fall, dass n für eine ganze Zahl kleiner als 8 steht, die Gruppe ausgewählt aus den allgemeinen Formeln (II-c-1) bis (II-c-6) an den restlichen 8 - n mit * gekennzeichneten Verknüpfungsstellen eine Flügelgruppe F trägt,
wobei
- F: für H, gegebenenfalls substituiertes C₁C₂₂-Alkyl, C₁-C₂₂-Halogenalkyl, C₁-C₂₂-Alkenyl, C₁-C₂₂-Alkoxy, C₁-C₂₂-Thioalkyl, C₁-C₂₂-Iminoalkyl, C₁-C₂₂-Alkoxycarbonyl, C₁-C₂₂-Alkoxycarbonyloxy, einen Rest einer aliphatischen C₁-C₂₂-Alkancarbonsäure oder der Acrylsäure, Halogen, Pseudohalogen, NO₂, eine Carboxyl-, eine Sulfonsäure- bzw. Sulfonat- oder eine Hydroxygruppe steht.

Ebenfalls bevorzugt kann im Rahmen der Erfindung
- M: für einen Steroidrest oder ein Derivat eines Steroidrestes stehen, insbesondere bevorzugt für einen Cholesterylrest oder ein Derivat des Cholesterylrestes der allgemeinen Formel (III-a) steht, 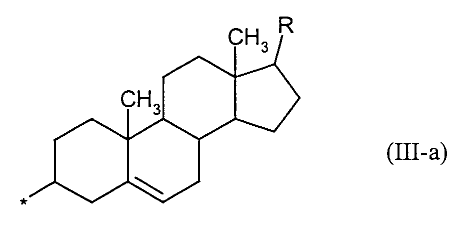 worin
R für H, gegebenenfalls substituiertes C₁-C₂₂-Alkyl, C₁-C₂₂-Halogenalkyl, C₁-C₂₂-Alkenyl, C₁-C₂₂-Alkoxy, C₁-C₂₂-Thioalkyl, C₁-C₂₂-Iminoalkyl, C₁-C₂₂-Alkoxycarbonyl, C₁-C₂₂-Alkoxycarbonyloxy, einen Rest einer aliphatischen C₁-C₂₂-Alkancarbonsäure oder der Acrylsäure, Halogen, Pseudohalogen, NO₂ eine Carboxyl-, eine Sulfonsäure- bzw. Sulfonat- oder eine Hydroxygruppe steht.

Die Anknüpfung der Steroidreste oder deren Derivate erfolgt bevorzugt über das C(3)-Atom des A-Ringes im Gonan-Grundgerüst

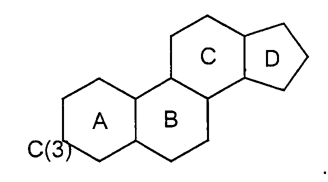

Steroidreste oder Derivate von Steroidresten können neben dem Cholesterylrest und dessen Derivaten beispielsweise solche der allgemeinen Formeln (III-b) bis (III-e) sein, 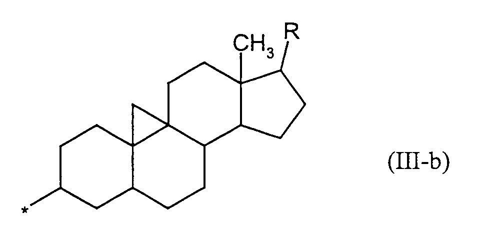 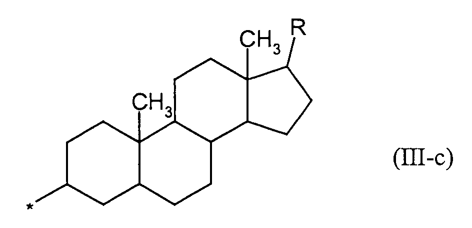 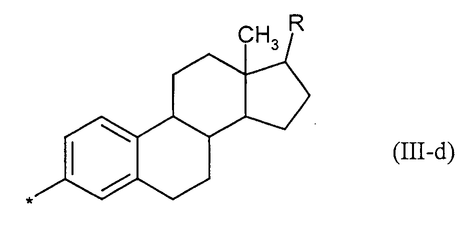 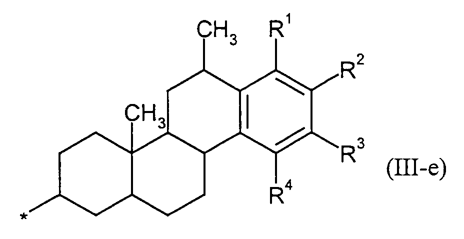 worin R, R¹, R², R³ und R⁴ unabhängig voneinander die vorangehend für R genannte Bedeutung haben können. Allerdings handelt es sich hierbei lediglich um eine beispielhafte, nicht jedoch abschließende Aufzählung.

In den allgemeinen Formeln (III-a) bis (III-e) sind die Konfigurationen an den einzelnen Stereozentren nicht eingezeichnet. Die vorliegende Erfmdung umfasst grundsätzlich alle bekannten Stereoisomeren sowie Gemische daraus, bevorzugt sind jedoch die natürlich vorkommenden Stereoisomeren sowie Gemische daraus.

Die erfindungsgemäßen 3,4-Alkylendioxythiophene können wenigstens ein chirales C-Atom im Alkylenrest A enthalten. In diesen Fällen sind im Rahmen der Erfindung unter erfindungsgemäßen 3,4-Alkylendioxythiophenen sowohl die reinen Stereoisomeren, Enantiomeren oder Diastereomeren, als auch beliebige Mischungen aus diesen zu verstehen.

Durch die Anwesenheit chiraler Verbindungen in flüssigkristallinen Phasen kann eine helixartige Überstruktur gebildet werden. Derartige Phasen können ferroelektrische Eigenschaften aufweisen.

Die erfindungsgemäßen 3,4-Alkylendioxythiophene können aus Hydroxyverbindungen der allgemeinen Formel (V), 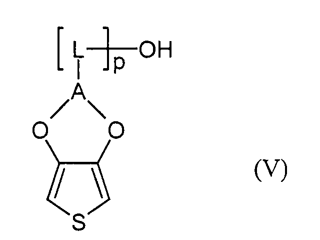 worin A, L und p oben genannte Bedeutung haben können,
nach bekannten Verfahren je nach Bedeutung von B und M durch Umsetzung mit entsprechenden Reaktanden hergestellt werden. Prinzipiell sind alle Verfahren unter Einsatz aller Reaktanden geeignet, die mit der Hydroxygruppe in Formel (V) die Knüpfung einer Ethergruppe (-0-) oder Oxycarbonylgruppe (-O-CO-) ermöglichen. Bei den bekannten Verfahren kann es sich je nach Bedeutung von B beispielsweise um Veresterungs- oder Veretherungsreaktionen handeln, jedoch kommen auch andere Reaktiontypen in Frage. Die Reaktanden können als gegenüber der Hydroxygruppe in Formel (V) reaktive funktionelle Gruppe beispielsweise eine Carbonsäuregruppe oder deren Derivat, eine Aldehydgruppe, Halogen oder andere enthalten. Beispielhaft seien hier Cholesterylchlorformiat, [(4'-Cyan-1,1'-biphenyl-4-yl)oxy]-carbonsäuren oder [(4'-Brom-1,1'-biphenyl-4-yl)oxy]carbonsäuren genannt. Geeignete Reaktanden, die nicht käuflich zu erwerben sind, können vor der Anknüpfung an die Verbindungen der Formel (V) aus Edukten enthaltend die Gruppierung B bzw. M oder aus Edukten, die durch ihre Verknüpfung erst die Gruppierungen B und M bilden, hergestellt werden. Es ist auch möglich B und M stufenweise anzuknüpfen, indem man die Hydroxyverbindungen der allgemeinen Formel (V) zunächst mit einem bifunktionellen Reaktanden umsetzt, der beispielsweise die Gruppierung B enthält, und das Zwischenprodukt nach Isolierung oder in situ mit einem weiteren beispielsweise die Gruppierung M enthaltenden Reaktanden zu den erfindungsgemäßen 3,4-Alkylendioxythiophenen umsetzt. Derartige Verfahren sind dem Fachmann ebenfalls bekannt. Als bifunktionelle Reaktanden seien beispielhaft α,ω-Dihalogenverbindungen, wie 1,6-Dibromhexan, genannt; als die Gruppierung M enthaltenden Reaktanden seien beispielhaft 4-(4-Alkoxyphenyl)phenol und 4-(4-Bromphenyl)phenol genannt.

Die Hydroxyverbindungen der Formel (V) können in einer sauer katalysierten Umetherungsreaktion aus Alkantriolen und 3,4-Dialkoxythiophenen hergestellt werden. Geeignete 3,4-Dialkoxythiophene hierfür sind insbesondere diejenigen mit kurzkettigen n-Alkoxygruppen, bevorzugt Methoxy-, Ethoxy- und n-Propoxy-Gruppen. Diese Arbeitsweise ist prinzipiell in Adv. Mater. **11** (1999), S. 1379 - 1381 beschrieben. Die bevorzugten Ausgangsverbindungen EDT-Methanol und Hydroxy-PDT können auch gemäß US-A 5.111.327 im Gemisch hergestellt werden. Es können aber auch die reinen Verbindungen eingesetzt werden, die z. B. durch eine chromatographische Trennung gemäß US-A 5.111.327 erhältlich sind. EDT-Methanol kann auch direkt in reiner Form gemäß Reynolds et al., Polym. Prepr. (Am. Chem. Soc., Div. Polym. Chem.) **38**(2), (1997), 320 mittels 2,3-Dibrompropylacetat hergestellt werden.

Die erfindungsgemäßen 3,4-Alkylendioxythiophene eignen sich hervorragend zur Herstellung von Polythiophenen.

Daher ist ebenfalls Gegenstand der vorliegenden Erfindung die Verwendung der erfindungsgemäßen 3,4-Alkylendioxythiophene oder Mischungen aus diesen zur Herstellung von Polythiophenen.

Weiterhin Gegenstand der Erfindung sind Polythiophene, dadurch gekennzeichnet, dass sie wiederkehrende Einheiten der allgemeinen Formel (IV) enthalten, 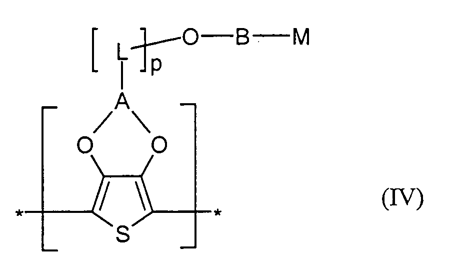 worin
A, L, p, M und B die oben genannte Bedeutung haben,
ausgenommen Polythiophene bestehend aus wiederkehrenden Einheiten der Formel (ii)

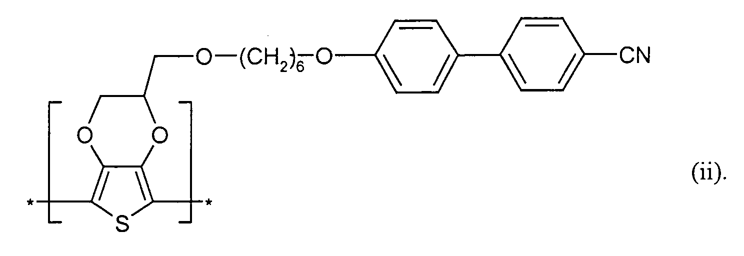

Im Rahmen der Erfmdung können dies Polythiophene sein, die gleiche oder verschiedene wiederkehrende Einheiten der allgemeinen Formel (IV) enthalten, wobei es im Falle verschiedener wiederkehrender Einheiten der allgemeinen Formel (IV) bei den Polythiophenen um Copolymere handelt. Je nach Anordnung der verschiedenen wiederkehrenden Einheiten der allgemeinen Formel (IV) kann es sich dabei um statistische, alternierende, Gradienten- oder Block-Copolymere handeln.

Bevorzugte erfindungsgemäße Polythiophene sind dadurch gekennzeichnet, dass sie wiederkehrende Einheiten der allgemeinen Formel (IV-a) und/oder (IV-b) enthalten, 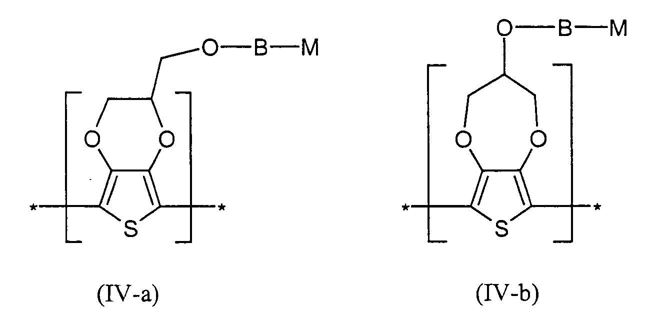 worin

M und B die oben genannte Bedeutung haben.

In diesen bevorzugten erfindungsgemäßen Polythiophenen können im Rahmen der Erfindung die wiederkehrenden Einheiten der allgemeinen Formel (IV-a) und (IV-b) in einem beliebigen Stoffmengenverhältnis enthalten sein. Besonders bevorzugt sind erfindungsgemäße Polythiophene die wiederkehrenden Einheiten der allgemeinen Formel (IV-a) mit einem Anteil von 65 bis 99,9 %, ganz besonders bevorzugt mit einem Anteil von 75 bis 99,5 % bezogen auf die Gesamtstoffmenge der wiederkehrenden Einheiten, enthalten, und die wiederkehrenden Einheiten der allgemeinen Formel (IV-b) besonders bevorzugt mit einem Anteil von 0,1 bis 35 %, ganz besonders bevorzugt mit einem Anteil von 0,1 bis 25 %, bezogen auf die Gesamtstoffmenge wiederkehrenden Einheiten enthalten, mit der Maßgabe, dass die Summe beider Anteile 100 % ergibt.

Unter dem Begriff Polythiophene sind im Rahmen der Erfindung bevorzugt alle Verbindungen umfasst, die mindestens 2 und höchstens 200, bevorzugt mindestens 2 und höchstens 50, wiederkehrende Einheiten der allgemeinen Formel (IV) enthalten.

Bevorzugt sind die erfindungsgemäßen Polythiophene neutral und halbleitend oder kationisch und elektrisch leitfähig. Für den Fall, dass es sich um kationische Polythiophene handelt, werden die positiven Ladungen der Polythiophen-Polykationen nicht in den Formeln (IV), (IV-a), (IV-b) dargestellt, da ihre genaue Zahl und ihre Position nicht einwandfrei feststellbar sind. Die Anzahl der positiven Ladungen ist jedoch mindestens 1 und höchstens die Gesamtzahl aller wiederkehrenden Einheiten im Polythiophen.

Zur Kompensation der positiven Ladung enthält die kationische Form der Polythiophene Anionen, vorzugsweise Polyanionen, als Gegenionen.

Als Polyanionen dienen vorzugsweise die Anionen von polymeren Carbonsäuren, wie Polyacrylsäuren, Polymethacrylsäure oder Polymaleinsäuren und polymeren Sulfonsäuren, wie Polystyrolsulfonsäuren und Polyvinylsulfonsäuren. Diese Polycarbon- und -sulfonsäuren können auch Copolymere von Vinylcarbon- und Vinylsulfonsäuren mit anderen polymerisierbaren Monomeren, wie Acrylsäureestern und Styrol, sein.

Besonders bevorzugt ist das Anion der Polystyrolsulfonsäure als Gegenion.

Das Molekulargewicht der die Polyanionen liefernden Polysäuren beträgt vorzugsweise 1 000 bis 2 000 000, besonders bevorzugt 2 000 bis 500 000. Die Polysäuren oder ihre Alkalisalze sind im Handel erhältlich, z.B. Polystyrolsulfonsäuren und Polyacrylsäuren.

Die Polythiophene enthaltend wiederkehrende Einheiten der allgemeinen Formel (IV) können durch chemische oder elektrochemische oxidative Polymerisation der erfindungsgemäßen 3,4-Alkylendioxythiophene hergestellt werden.

Weiterhin Gegenstand der vorliegenden Erfindung ist somit ein Verfahren zur Herstellung von Polythiophenen enthaltend wiederkehrende Einheiten der allgemeinen Formel (IV), ausgenommen Polythiophene bestehend aus wiederkehrenden Einheiten der Formel (ii), dadurch gekennzeichnet, dass Verbindungen der allgemeinen Formel (I), 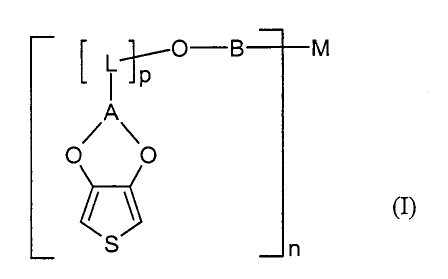 worin
A, L, p, M und B die oben genannte Bedeutung haben,
elektrochemisch oxidativ polymerisiert werden.

Die elektrochemische oxidative Polymerisation der erfindungsgemäßen 3,4-Alkylendioxythiophene kann bei Temperaturen von -78°C bis zum Siedepunkt des eingesetzten Lösungsmittels bzw. erfindungsgemäßen 3,4-Alkylendioxythiophen vorgenommen werden. Bevorzugt wird bei Temperaturen von -20°C bis 60°C elektrolysiert.

Die Reaktionszeiten betragen in Abhängigkeit vom verwendeten Monomer, dem verwendeten Elektrolyten, der gewählten Elektrolysetemperatur und der angewendeten Stromdichte 1 Minute bis 24 Stunden.

Sofern die erfindungsgemäßen 3,4-Alkylendioxythiophene unter den Elektrolysebedingungen flüssig sind, kann die Elektropolymerisation in An- oder Abwesenheit von unter den Elektrolysebedingungen inerten Lösungsmitteln vorgenommen werden; die Elektropolymerisation von unter den Elektrolysebedingungen festen erfindungsgemäßen 3,4-Alkylendioxythiophenen wird in Gegenwart von unter den Elektrolysebedingungen inerten Lösungsmitteln durchgeführt. In bestimmten Fällen kann es vorteilhaft sein, Lösungsmittelgemische einzusetzen und/oder den Lösungsmitteln Lösungsvermittler (Detergentien) zuzusetzen.

Als unter den Elektrolysebedingungen inerte Lösungsmittel seien beispielsweise genannt: Wasser; Alkohole wie Methanol und Ethanol; Ketone wie Acetophenon; halogenierte Kohlenwasserstoffe wie Methylenchlorid, Chloroform, Tetrachlorkohlenstoff und Fluorkohlenwasserstoffe; Ester wie Ethylacetat und Butylacetat; Kohlensäureester wie Propylencarbonat; aromatische Kohlenwasserstoffe wie Benzol, Toluol, Xylol; aliphatische Kohlenwasserstoffe wie Pentan, Hexan, Heptan und Cyclohexan; Nitrile wie Acetonitril und Benzonitril; Sulfoxide wie Dimethylsulfoxid; Sulfone wie Dimethylsulfon, Phenylmethylsulfon und Sulfolan; flüssige aliphatische Amide wie Methylacetamid, Dimethylacetamid, Dimethylformamid, Pyrrolidon, N-Methylpyrrolidon, Caprolactam, N-Methylcaprolactam; aliphatische und gemischt aliphatisch-aromatische Ether wie Diethylether und Anisol; flüssige Harnstoffe wie Tetramethylharnstoff oder N-N-Dimethyl-imidazolidinon. Die Polymerisation kann auch aus lyotroper flüssigkristalliner Phase heraus erfolgen.

Für die Elektropolymerisation werden die erfindungsgemäßen 3,4-Alkylendioxythiophene bzw. deren Lösungen mit Elektrolytzusätzen versetzt. Als Elektrolytzusätze werden vorzugsweise freie Säuren oder übliche Leitsalze verwendet, die eine gewisse Löslichkeit in den verwendeten Lösungsmitteln aufweisen. Als Elektrolytzusätze haben sich z.B. bewährt: freie Säuren wie p-Toluolsulfonsäure, Methansulfonsäure, ferner Salze mit Alkansulfonat-, aromatischen Sulfonat-, Tetrafluoroborat-, Hexafluorophosphat-, Perchlorat-, Hexafluoroantimonat-, Hexafluoroarsenat- und Hexachloroantimonat-Anionen und Alkali-, Erdalkali- oder gegebenenfalls alkylierten Ammonium-, Phosphonium-, Sulfonium- und Oxonium-Kationen. Gegenebenfalls können bei der elektrochemischen Polymerisation auch die o.g. polymeren Gegenionen der Elektrolyselösung oder den erfindungsgemäßen 3,4-Alkylendioxythiophenen als Elektrolytzusätze oder Leitsalze zugegeben.

Die Konzentrationen der erfindungsgemäßen 3,4-Alkylendioxythiophene können zwischen 0,01 und 100 Gew.-% (100 Gew.-% nur bei flüssigem Thiophen) liegen; bevorzugt betragen die Konzentrationen 0,1 bis 5 Gew.-%.

Die Elektropolymerisation kann diskontinuierlich oder kontinuierlich durchgeführt werden.

Die Stromdichten für die Elektropolymerisation können in weiten Grenzen schwanken; üblicherweise wird mit Stromdichten von 0,0001 bis 100 mA/cm², vorzugsweise 0,01 bis 40 mA/cm² gearbeitet. Bei diesen Stromdichten stellen sich Spannungen von etwa 0,1 bis 50 V ein.

Ebenfalls Gegenstand der vorliegenden Erfindung ist ein Verfahren zur Herstellung von Polythiophenen, enthaltend wiederkehrende Einheiten der allgemeinen Formel (IV), dadurch gekennzeichnet, dass Verbindungen der allgemeinen Formel (I), 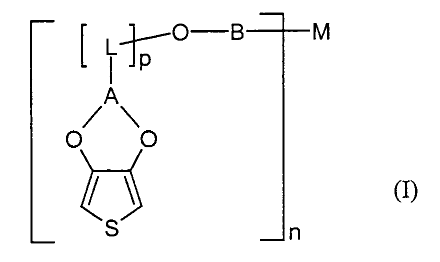 worin
A, L, p, M und B die oben genannte Bedeutung haben,
chemisch oxidativ polymerisiert werden.

Die oxidative chemische Polymerisation der erfindungsgemäßen 3,4-Alkylendioxythiophene wird je nach verwendetem Oxidationsmittel und gewünschter Reaktionszeit im allgemeinen bei Temperaturen von -10°C bis 250°C, bevorzugt bei Temperaturen von 20°C bis 200°C, vorgenommen.

Als Lösungsmittel für die erfindungsgemäßen 3,4-Alkylendioxythiophene und/oder Oxidationsmittel seien vor allem folgende unter den Reaktionsbedingungen inerten organischen Lösungsmittel genannt: aliphatische Alkohole wie Methanol, Ethanol, i-Propanol und Butanol; aliphatische Ketone wie Aceton und Methylethylketon; aliphatische Carbonsäureester wie Essigsäureethylester und Essigsäurebutylester; aromatische Kohlenwasserstoffe wie Toluol und Xylol; aliphatische Kohlenwasserstoffe wie Hexan, Heptan und Cyclohexan; Chlorkohlenwasserstoffe wie Dichlormethan und Dichlorethan; aliphatische Nitrile wie Acetonitril, aliphatische Sulfoxide und Sulfone wie Dimethylsulfoxid und Sulfolan; aliphatische Carbonsäureamid wie Methylacetamid und Dimethylformamid; aliphatische und araliphatische Ether wie Diethylether und Anisol. Weiterhin kann auch Wasser oder Gemische aus Wasser mit den vorgenannten organischen Lösungsmitteln als Lösungsmittel verwendet werden. Die Polymerisation kann auch aus lyotroper flüssigkristalliner Phase heraus erfolgen.

Als Oxidationsmittel werden die dem Fachmann bekannten für die oxidative Polymerisation von Thiophenen geeigneten Oxidationsmittel verwendet; diese sind beispielsweise in G. Koßmehl, Makromol. Chem., Macromol. Symp. 4, 45-64 (1986)) beschrieben. Bevorzugt sind aus praktischen Gründen preiswerte und leicht handhabbare Oxidationsmittel wie Eisen-III-Salze anorganischer Säuren, wie beispielsweise FeCl₃, Fe(ClO₄)₃ oder Fe₂(SO₄)₃, und die Eisen-III-Salze organischer Säuren und organische Reste aufweisender anorganischer Säuren, ferner H₂O₂ K₂Cr₂O₇, Alkaliund Ammoniumperoxodisulfate, Alkaliperborate, Kaliumpermanganat, Kupfersalze, wie Kupfertetrafluoroborat oder Cer(IV)-Salze bzw. CeO₂. Gegebenenfalls kann die Anwesenheit katalytischer Mengen an Metallionen, wie Eisen-, Cobalt-, Nickel-, Kupfer, Molybdän- oder Vanadiumionen hilfreich sein.

Für die oxidative Polymerisation der erfindungsgemäßen 3,4-Alkylendioxythiophene und gegebenenfalls anschließende Oxidation der Poly-3,4-Alkylendioxythiophene werden theoretisch je Mol Thiophen ungefähr 2,25 Äquivalente Oxidationsmittel benötigt (siehe z.B. J. Polym. Sc. Part A Polymer Chemistry Vol. 26, S. 1287 (1988)). Es können aber auch niedrigere oder höhere Äquivalente an Oxidationsmittel eingesetzt werden. Im Rahmen der Erfindung wird je Mol Thiophen bevorzugt ein Äquivalent oder mehr, besonders bevorzugt 2 Äquivalente oder mehr Oxidationsmittel eingesetzt.

Als Eisen-III-Salze organische Reste aufweisender anorganischer Säuren seien beispielsweise die Eisen-III-Salze der Schwefelsäurehalbester von C₁-C₂₀-Alkanolen, z.B. das Fe-III-Salz des Laurylsulfates genannt.

Als Eisen-III-Salze organischer Säuren seien beispielsweise genannt: die Fe-III-Salze von C₁-C₂₀-Alkansulfonsäuren, wie der Methan- und der Dodecansulfonsäure, aliphatischen C₁-C₂₀-Carbonsäuren wie der 2-Ethylhexylcarbonsäure, aliphatischen Perfluorcarbonsäuren, wie der Trifluoressigsäure und der Perfluoroctansäure, aliphatischen Dicarbonsäuren, wie der Oxalsäure und vor allem von aromatischen, gegebenenfalls durch C₁-C₂₀-Alkylgruppen substituierten Sulfonsäuren wie der Benzolsulfonsäure, p-Toluolsulfonsäure und der Dodecylbenzolsulfonsäure und Cycloalkansulfonsäuren wie Camphersulfonsäure.

Es können auch Gemische dieser vorgenannten Fe-III-Salze organischer Säuren eingesetzt werden.

Außerdem können als Gegenionen zu den erfindungsgemäßen Polythiophenen, für den Fall, dass es sich um kationische Polythiophene handelt, die gegebenenfalls vorhandenen Anionen des verwendeten Oxidationsmittels dienen, so dass im Falle der chemischen oxidativen Polymerisation eine Zugabe zusätzlicher Gegenionen nicht zwingend erforderlich ist.

In bevorzugten Ausführungsformen der erfindungsgemäßen Verfahren werden als Verbindungen der allgemeinen Formel (I) oder Mischungen aus diesen, Verbindungen mit einer Struktur der allgemeinen Formel (I-a) und/oder (I-b), 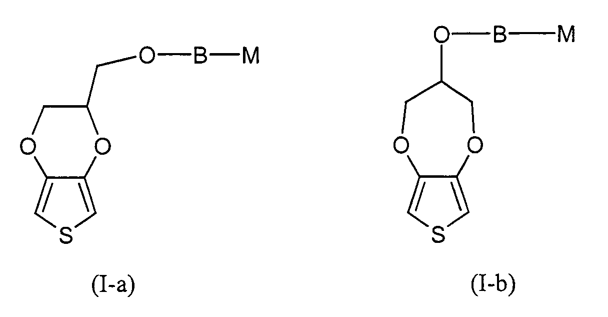 worin

B und M die oben genannte Bedeutung haben, eingesetzt.

In besonders bevorzugten Ausführungsformen des erfindungsgemäßen Verfahrens wird ein Gemisch aus 3,4-Alkylendioxythiophenen der allgemeinen Formeln (I-a) und (I-b), worin B und M die oben genannte Bedeutung haben, eingesetzt.

In diesen Gemischen können im Rahmen der Erfindung die 3,4-Alkylendioxythiophene der allgemeinen Formel (I-a) und (I-b) in einem beliebigen Stoffinengenverhältnis enthalten sein. Bevorzugt sind die 3,4-Alkylendioxythiophene der allgemeinen Formel (I-a) mit einem Anteil von 65 bis 99,9 %, besonders bevorzugt mit einem Anteil von 75 bis 99,5 % bezogen auf die Gesamtstoffmenge der Thiophene, enthalten, und die 3,4-Alkylendioxythiophenen der allgemeinen Formel (I-b) sind bevorzugt mit einem Anteil von 0,1 bis 35 %, besonders bevorzugt mit einem Anteil von 0,1 bis 25 %, bezogen auf die Gesamtstoffmenge der Thiophene enthalten, mit der Maßgabe, dass die Summe beider Anteile 100 % ergibt. Dabei können die Stoffmengenanteile der 3,4-Alkylendioxythiophene der allgemeinen Formeln (I-a) und (I-b) den Stoffinengenanteilen der wiederkehrenden Einheiten der allgemeinen Formeln (VI-a) und (VI-b) in den resultierenden erfindungsgemäßen Polythiophenen entsprechen, können aber auch von diesen verschieden sein.

C₁-C₅-Alkylenreste können im Rahmen der Erfindung Methylen, Ethylen, n-Propylen, n-Butylen oder n-Pentylen sein, lineare oder cyclische C₁-C₂₀-Alkylenreste darüberhinaus n-Hexylen, n-Heptylen, n-Octylen, n-Nonylen, n-Decylen, n-Undecylen, n-Dodecylen, n-Tridecylen oder n-Tetradecylen, n-Hexadecylen, n-Octadecylen oder n-Eicosylen, Cyclopentylen, Cyclohexylen, Cycloheptylen, Cyclooctylen, Cyclononylen oder Cyclodecylen. Lineare, verzweigte oder cyclische C₁-C₂₂-Alkylreste können im Rahmen der Erfindung beispielsweise Methyl, Ethyl, n-oder iso-Propyl, n-, iso-, sec- oder tert-Butyl, n-Pentyl, 1-Methylbutyl, 2-Methylbutyl, 3-Methylbutyl, 1-Ethylpropyl, 1,1-Dimethylpropyl, 1,2-Dimethylpropyl, 2,2-Dimethylpropyl, n-Hexyl, n-Heptyl, n-Octyl, 2-Ethylhexyl, n-Nonyl, n-Decyl, n-Undecyl, n-Dodecyl, n-Tridecyl oder n-Tetradecyl, n-Hexadecyl, n-Octadecyl oder n-Eicosyl, n-Docosyl, Cyclopentyl, Cyclohexyl, Cycloheptyl, Cyclooctyl, Cyclononyl oder Cyclodecyl sein, wobei cylische Alkylreste mindestens C₅-Cycloalkylreste sind. C₅-C₂₀-Arylenreste können beispielsweise Phenylen, Naphthylen, Biphenylen, Fluorenylen, Indenylen, Cyclopentadienylen oder Anthracenylen und C₆-C₂₀-Aralkylenreste beispielsweise o-, m-, p-Tolylen, Benzylen, 2,3-, 2,4-, 2,5-, 2,6-, 3,4-, 3,5-Xylylen oder Mesitylen sein. C₂-C₂₀-Heteroarylenreste können auf allen heteroaromatischen Ringsystemen basieren, die zusätzlich zu den C-Atomen noch ein bis drei Heteroatome ausgewählt aus N, O oder S im heteroaromatischen Ring- oder Ringsystem aufweisen, wie beispielsweise Pyrrol. Thiophen, Furan, Pyridin, Indol, Carbazol, Pyrazol, Imidazol oder Thiazol. C₂-C₂₀₀-Oligo- oder -Polyethergruppen können im Rahmen der Erfindung solche mit ein bis 50 O-Atomen in der Oligo- bzw. Polyetherkette, C₂-C₈₀-Oligo- oder -Polyethergruppen solche mit ein bis 20 O-Atomen, sein, wobei die kleinste Oligoethergruppe mit zwei C-Atomen eine Dimethylenethergruppe (-CH₂-O-CH₂-) ist. C₁-C₂₂ Halogenalkyl, C₁-C₂₂-Alkenyl, C₁-C₂₂-Alkoxy, C₁-C₂₂-Thioalkyl, C₁-C₂₂-Iminoalkyl, C₁-C₂₂-Alkoxycarbonyl, C₁-C₂₂-Alkoxycarbonyloxy sowie Reste von aliphatischen C₁-C₂₂-Alkancarbonsäuren können sich von vorangehend aufgeführten C₁-C₂₂-Alkylresten durch entsprechende Substitution eines H-Atoms durch die funktionelle Gruppe ableiten. Halogen können beispielsweise Fluor, Chlor, Brom und Iodreste, Pseudohalogen beispielsweise Cyan, Thiocyan, Isocyan oder Isothiocyanreste sein. Die vorangehende Aufzählung dient der beispielhaften Erläuterung der Erfindung und ist nicht als abschließend zu betrachten.

Als gegebenenfalls vorhandene Substituenten beispielsweise für C₁-C₂₂-Alkyl kommen beispielsweise Halogen, Pseudohalogen, Keto-, Aldehyd-, Amino-, Hydroxy-, Nitro-, Thiohydroxy-, Carboxyl-, Carboxylat-, Sulfonsäure- oder Sulfonatgruppen in Frage

Kombiniert man wie im Falle der vorliegenden Erfindung das Prinzip der mesogenen Gruppen mit Polymeren, so kann man flüssigkristalline Polymere erhalten. Es können jedoch auch, bedingt durch den molekularen Ordnungszustand, Polymere mit verbesserten Eigenschaften, wie z.B. höherer Leitfähigkeit, entstehen, die keine flüssigkristallinen Eigenschaften zeigen.

Die erfindungsgemäßen Polythiophene eignen sich als Bestandteile in elektrischen oder elektronischen Bauteilen, lichtemittierenden Bauteilen, insbesondere organischen elektrischen oder elektronischen Bauteilen bzw. lichtemittierenden Bauteilen, z.B. Leuchtelementen, Photozellen oder organischen Transistoren. Des weiteren können sie zur antistatischen Beschichtung, z.B. zur Ausrüstung von Kunststofffolien für die Verpackung elektronischer Bauteile und für Reinraumverpackungen, zur antistatischen Ausrüstung von Kathodenstrahlröhren oder zur antistatischen Ausrüstung von photographischen Filmen, in der Optoelektronik, z.B. als transparente Heizung, als transparente Elektroden, als Leiterplatten oder für elektrisch einfärbbare Fensterscheiben, oder in der Solarenergie-Technik verwendet werden.

Gegenstand der vorliegenden Erfindung ist daher ebenfalls die Verwendung der erfindungsgemäßen Polythiophene als Bestandteil in elektrischen oder elektronischen Bauteilen, lichtemittierenden Bauteilen, zur antistatischen Beschichtung, in der Optoelektronik oder in der Solarenergie-Technik.

Die Leitfähigkeit beispielsweise von Bestandteilen in elektrischen oder elektronischen Bauteilen, lichtemittierenden Bauteilen oder antistatischen Beschichtungen aus den erfindungsgemäßen Polythiophenen kann durch eine Temperaturbehandlung (Temperung) weiter erhöht werden. Dabei können unter Umständen flüssigkristalline Phasen durchlaufen werden. Die Temperung erfolgt bevorzugt bei Temperaturen von 80°C bis 300°C, besonders bevorzugt von 100°C bis 250°C, für einen Zeitraum von 15 Minuten bis 6 Stunden, besonders bevorzugt 30 Minuten bis 4 Stunden. Bei erfindungsgemäßen Polythiophenen, die gemäß chemischer oxidativer Polymerisation hergestellt wurden, sollte die Temperung bevorzugt nach Auswaschen überschüssiger Reaktanden, wie beispielsweise Oxidationsmittel oder überschüssigen Monomers, mit einem geeigneten Lösungsmittel, bevorzugt Wasser oder Alkoholen, erfolgen.

Bevorzugt werden die erfindungsgemäßen Polythiophene in Form leitfähiger Schichten bzw. Beschichtungen in elektrischen oder elektronischen Bauteilen, lichtemittierenden Bauteilen, zur antistatischen Beschichtung, in der Optoelektronik oder in der Solarenergie-Technik eingesetzt. Diese leitfähigen Schichten werden vorzugsweise mittels *in situ*-Polymerisation der entsprechenden erfindungsgemäßen 3,4-Alkylendioxythiophene und einem oder mehreren Oxidationsmitteln gegebenenfalls in Anwesenheit zusätzlicher Gegenionen hergestellt. Der Begriff der *in situ-*Polymerisation ist dem Fachmann bekannt.

In bevorzugten Ausführungsformen werden die leitfähigen Schichten zur Erhöhung der Leitfähigkeit einer Temperaturbehandlung (Temperung) unterzogen.

Die leitfähigen Schichten können beispielsweise hergestellt werden, indem die erfindungsgemäßen 3,4-Alkylendioxythiophene, Oxidationsmittel und gegebenenfalls Gegenionen, zusammen oder nacheinander gegebenenfalls in Form von Lösungen, auf die zu beschichtende Unterlage aufgebracht und chemisch oxidativ zu den erfindungsgemäßen Polythiophenen polymerisiert werden und gegebenenfalls vorhandenes Lösungsmittel vor, während oder nach der Polymerisation entfernt wird.

Die Aufbringung der Lösungen auf die zu beschichtende Unterlage kann nach bekannten Verfahren, z.B. durch Tränkung, Gießen, Auftropfen, Spritzen, Aufsprühen, Aufrakeln, Bestreichen oder Bedrucken erfolgen.

### Beispiele

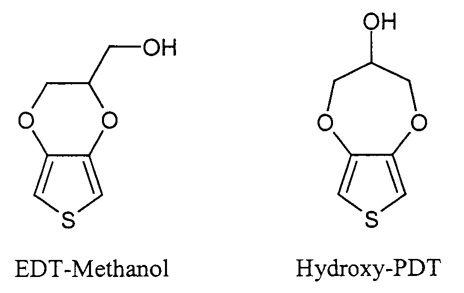

Das im Folgenden verwendete EDT-Methanol/Hydroxy-PDT-Gemisch wurde gemäß US-A 5.111.327 hergestellt. Cholesterylchlorformiat ist kommerziell erhältlich; [(4'-Cyan-1,1'-biphenyl-4-yl)oxy]essigsäure, [(4'-Cyan-1,1'-biphenyl-4-yl)oxy]buttersäure und [(4'-Cyan-1,1'-biphenyl-4-yl)oxy]valeriansäure sowie [(4'-Brom-1,1'-biphenyl-4-yl)oxy]valeriansäure wurden gemäß J. Am. Chem. Soc. 119 (1997), S. 5825-5826 hergestellt.

### Beispiel 1:

2,46 g eines Gemisches aus 80 % EDT-Methanol und 20 % Hydroxy-PDT werden (entsprechend 11,5 mmol EDT-Methanol) werden in trockenem Methylenchlorid gelöst und eisgekühlt. 5,68 g (12,6 mmol) Cholesterylchlorformiat in trockenem Methylenchlorid werden zu dieser gekühlten Lösung zugegeben. Anschließend werden langsam 1,40 g (13,8 mmol) Triethylamin zugetropft und das Eisbad entfernt. Die Reaktionsmischung wird für 30 h bei Raumtemperatur (23°C) gerührt, anschließend werden nochmals 2,64 g (5,9 mmol) Cholesterylchlorformiat sowie 0,63 g (6,2 mmol) Triethylamin in Methylenchlorid langsam hinzugefügt. Nach weiteren 72 h Reaktionszeit wird die Lösung je drei Mal mit 1 M (molarer) HCl und gesättigter NaHCO₃-Lösung ausgeschüttelt. Die organische Phase wird mit MgSO₄ getrocknet und das Lösungsmittel am Rotationsverdampfer bei 20 mbar entfernt.

Das Rohprodukt wird durch zweimalige Säulenchromatographie gereinigt (Laufmittel: 1. CHCl₃, 2. Petrolether, mit CHCl₃ eluiert). Das Produkt wird aus Aceton umkristallisiert, man erhält 0,93 g (I-a1) (im Gemisch mit 2 % (I-b1); Prozentangaben wenn nichts anderes erwähnt bezogen auf die Stoffmenge) als farbloses Pulver. 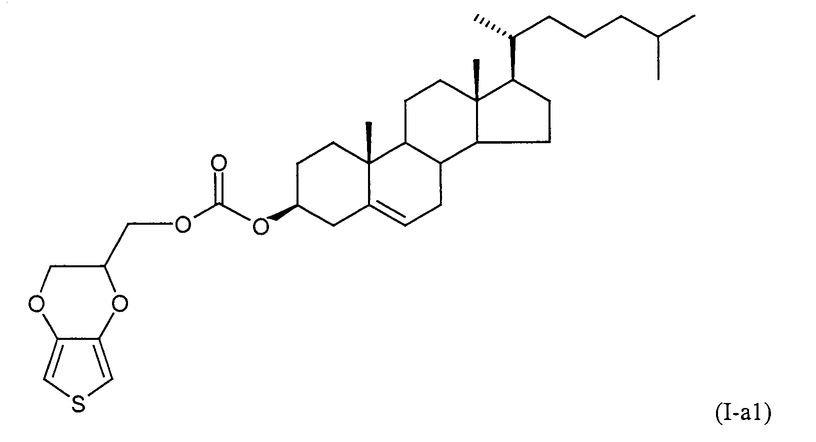 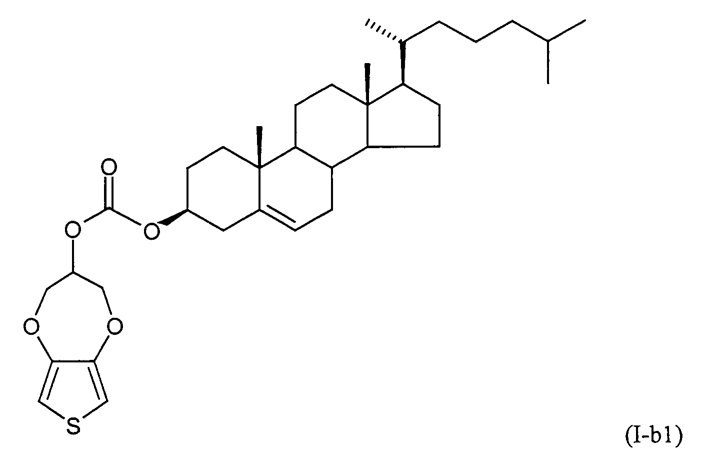 **IR** ν⁻¹ [cm⁻¹] = 2939, 2902, 2867, 2848; 1740
^{**1**}**H-NMR** (200 MHz, CDCl₃): δ [ppm] = 6,48 (s, 2% 2H, Nebenprodukt I-b1); 6,35, 6,32 (2*d, je 98 % 1H, ⁴*J*_{CH-CH}: 3,91 Hz); 5,38 (d, 1H); 4,62 - 4,15 (m, 5H); 4,26 (dd, 1H, *J*_{CH-CH}: 4,05 Hz); 2,38 (d, 2H); 2,17 - 0,73 (m, 38H); 0,99 (s, 3H); 0,89 (d, 3H); 0,84 (d, 6H); 0,66 (s, 3H)
^{**13**}**C-NMR** (50,3 MHz, CDCl₃): δ [ppm] = 154,16; 141,19, 140,93; 139,20; 123,14; 100,18, 99,98; 78,58; 71,26, 65,48, 65,18; 56,70; 56,16; 50,02; 42,34; 39,74; 39,53; 37,97; 36,85; 36,55; 36,21; 35,80; 31,92; 31,86; 28,23; 28,03; 27,66; 24,29; 23,85; 22,82; 22,59; 19,27, 18,74; 11,88
**MS** (FD, 5 kV): m/z = 585,5 [M+H]⁺

### Phasenübergänge (bestimmt mit dem Polarisationsmikroskop):

Schmp. 131,9°C, Klarpunkt 137,5 °C (beim 1. Heizen)
K 131,9 FC 137,5 I (K = kristallin, FC = flüssigkristallin, I = isotrop flüssig; die Zahlenwerte zwischen den Phasenbezeichnungen geben die Übergangstemperatur in °C an, z.B. K 131,9 FC = Übergang von der kristallinen zur flüssigkristallinen Phase bei 131,9°C)

### Beispiel 2:

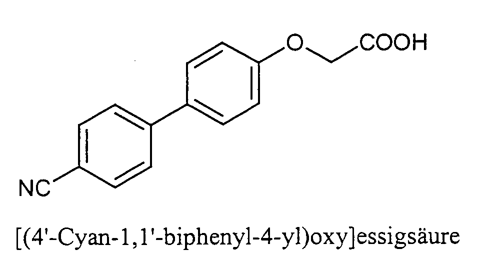

1,73 g eines Gemisches aus 80 % EDT-Methanol und 20 % Hydroxy-PDT (entsprechend 8,08 mmol EDT-Methanol), 1,64 g N,N'-Dicyclohexylcarbodiimid und 0,13 g 4-(Dimethylamino)pyridin werden in 100 ml CH₂Cl₂ gelöst und im Eisbad auf ca. 0°C gekühlt. Dazu werden unter Rühren und Eiskühlung 2,00 g [(4'-Cyan-1,1'-biphenyl-4-yl)oxy]essigsäure innerhalb 1 h portionsweise hinzugefügt. Danach wird die Reaktion für 42,5 h bei 23°C weitergeführt. Anschließend wird der gebildete N,N'-Dlcyclohexylharnstoff abfiltriert. Das Filtrat wird nacheinander je zweimal mit 200 ml 1 M HCl, 200 ml gesättigter NaHCO₃-Lösung sowie 200 ml gesättigter NaCl-Lösung ausgeschüttelt. Die organische Phase wird über MgSO₄ getrocknet und nach Abfiltrieren des Magnesiumsulfats am Rotationsverdampfer vom Lösungsmittel befreit. Man erhält 3,71 g Rohprodukt, das säulenchromatographisch mit n-Hexan/Ethylacetat 3:2 gereinigt wird. Vom reinen Ester des EDT-Methanols (I-a2) werden 0,39 g als farblose Kristalle erhalten. 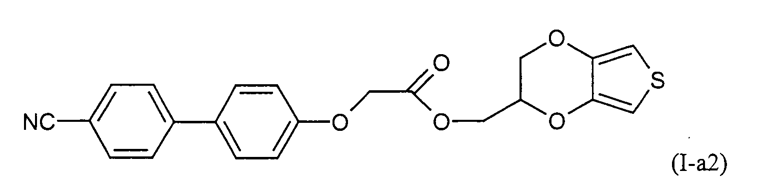 **IR** ν⁻¹ [cm⁻¹] = 2992, 2979, 2942, 2909, 2226, 1751 und 1743 , 1604, 1582 und 1495
^{**1**}**H-NMR** (500 MHz, DMSO-d₆): δ (ppm) = 7,87 (d, 2H, ³*J*_{CH-CH}: 8,51 Hz,); 7,81 (d, 2H ³*J*_{CH-CH}: 8,51 Hz); 7,68 (d, 2H, ³*J*_{CH-CH}: 8,83 Hz); 7,06 (d, 2H, ³*J*_{CH-CH}: 8,83 Hz); 6,61 (d, 1H, ⁴*J*_{CH-CH}: 3,78 Hz,); 6,58 (d, 1H, ⁴*J*_{CH-CH}: 3,78 Hz); 4,93 (s, 2H); 4,53 - 4,32 (m, 3H); 4,26 (dd, 1H, *J*_{CH-CH}: 11,67 Hz); 3,96 (dd, 1H, *J*_{CH-CH}: 11,67 Hz).
^{**13**}**C-NMR** (125,75 MHz, DMSO-d₆): δ (ppm) = 166,69; 156,38; 142,32; 139,28, 139,17, 130,99; 129,45; 126,53; 125,20; 117,19; 113,45; 107,52; 98,24, 98,20; 69,49; 63,03, 62,74; 60,70.

| | | | |
|---|---|---|---|
| MS (EI, 70 eV): m/z = | 407 | M⁺ | 100 % |
| | 208 | C₁₄H₁₀NO⁺ | 24,3 % |
| | 178 | C₁₃H₈N⁺ | 49,1 % |
| | 155 | C₇H₇O₂S+ | 22,5 % |

### Schmelzbereich: 125,2 - 127,7 °C (am Polarisationsmikroskop beobachtet).

### Beispiel 3:

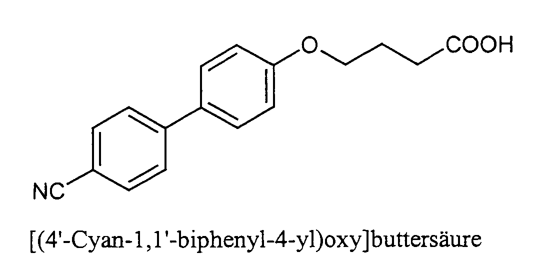

1,06 g eines Gemisches aus 80 % EDT-Methanol und 20 % Hydroxy-PDT (entsprechend 6,19 mmol; 4,95 mmol bezogen auf die Hauptkomponente EDT-Methanol), 1,04 g (5,04 mmol) N.N'-Dicyclohexylcarbodiimid und 0,15 g (1,22 mmol) 4-(Dimethylamino)- pyridin werden in 50 ml CH₂Cl₂ gelöst und in einem Eisbad abgekühlt. 1,42 g (5,05 mmol) [(4'-Cyan-1,1'-biphenyl-4-yl)oxy]-buttersäure (siehe Formel) werden portionsweise unter Rühren zu der eisgekühlten Lösung hinzugefügt. Nach 1 h wird die Reaktion bei Raumtemperatur fortgeführt. Nach weiteren 64 h wird der gebildete farblose Niederschlag (N,N'-Dicyclohexylharnstoff) abfiltriert. Die klare, farblose Lösung wird nacheinander mit je zwei Mal 75 ml 1 N HCl, 75 ml gesättigter NaHCO₃-Lösung sowie 75 ml gesättigter NaCl-Lösung ausgeschüttelt. Die klare organische Phase wird über MgSO₄ getrocknet, das Trockenmittel abfiltriert und das Lösungsmittel am Rotationsverdampfer entfernt. Man erhält 2,38 g Rohprodukt.

Die Aufreinigung erfolgt durch säulenchromatographische Trennung. Laufmittel: n-Hexan/ Ethylacetat 3:2. '
Ausbeute (I-a3)/(I-b3): 1,25 g farbloses Pulver (56,8 % der Theorie) 2 Isomere: ca. 95 % (I-a3) und 5 % (I-b3)

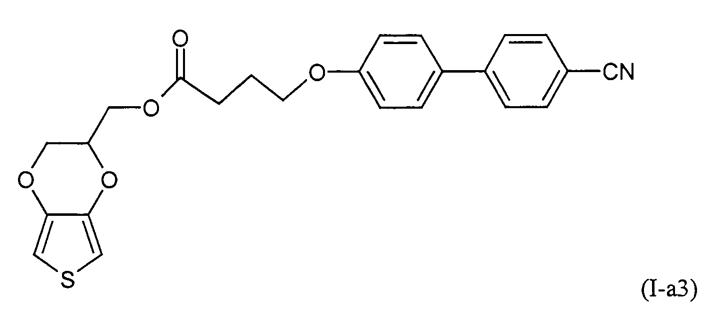

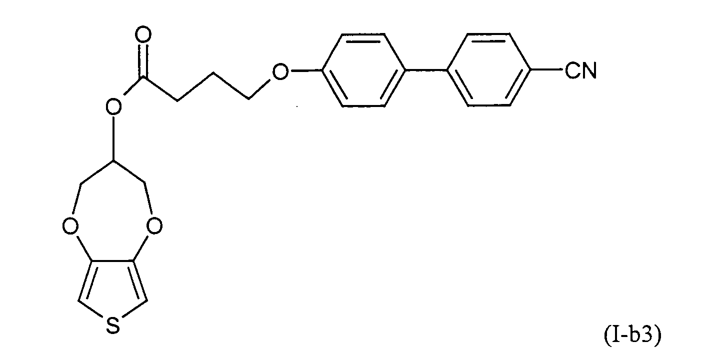

### Daten für (I-a3)/(I-b3), Stoffmengen-Verhältnis 95:5

**IR** ν⁻¹ [cm⁻¹] = 3117; 2969, 2947, 2923, 2905, 2884; 2222; 1734 1597, 1582, 1486; 827,815
^{**1**}**H-NMR** (500 MHz, DMSO-d₆): δ [ppm] = 7,86 (d, 2H); 7,81 (d, 2H); 7,68 (d, 2H, ³*J*_{CH-CH}: 8,83 Hz); 7,03 (d, 2H, ³*J*_{CH-CH}: 8,83 Hz); 6,79 (s, 5 % 2H, (I-b3)); 6,58 (s, 95 % 2H); 4,47 - 4,38 (m, 1H); 4,36 - 4,23 (m, 3H); 4,15 (d, 5 % 4H, (I-b3)); 4,05 (t, 2H); 4,00 (dd, 1H); 2,55 (t, 2H); 2,02 (tt , 2H)
^{**13**}**C-NMR** (125,75 MHz, DMSO-d₆): δ [ppm] = 172,46; 159,23; 144,36; 141,20, 141,11; 132,90; 130,58; 128,44; 126,97; 119,13; 115,23; 109,25; 100,08; 71,46; 66,72; 65,10; 62,24; 30,08; 24,22

| | | | |
|---|---|---|---|
| **MS** (EI, 70 eV): m/z = | 435 | M⁺ | 39,8 % |
| | 264 | C₁₇H₁₄NO₂ ⁺ | 12,6 % |
| | 241 | C₁₁H₁₃O₄S⁺ | 44,5 % |
| | 195 | C₁₃H₈NO⁺ | 34,3 % |
| | 155 | C₇H₇O₂S⁺ | 100 % |

### Phasenübergänge (bestimmt mit dem Polarisationsmikroskop):

Schmelzbereich: 119,8 - 121,9°C (beim 1. Aufheizen)isotrop bei 118,8°C beim 2. Heizen
K 116,9 FC 117,0 I (beim 3. Heizen)

### Beispiel 4:

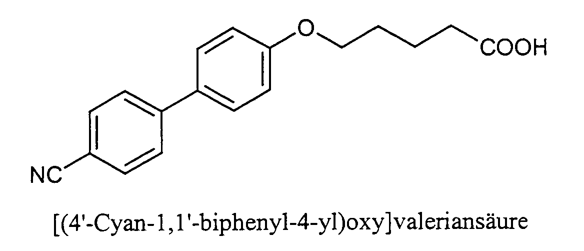

2,20 g eines Gemisches aus 80 % EDT-Methanol und 20 % Hydroxy-PDT (entsprechend 12,9 mmol; 10,3 mmol bezogen auf die Hauptkomponente EDT-Methanol), 2,10 g (10,2 mmol) N.N'-Dicyclohexylcarbodiimid und 1 Spatelspitze 4-(Dimethylamino)pyridin werden in 80 ml CH₂Cl₂ gelöst und in einem Eisbad abgekühlt. 3,00 g (10,2 mmol) [(4'-Cyan-1,1'-biphenyl-4-yl)oxy]valeriansäure (siehe Formel) werden spatelweise unter Rühren zu der kalten (0°C) Lösung hinzugefügt. Reste von [(4'-Cyan-1,1'-biphenyl-4-yl)oxy]valeriansäure werden in 20 ml CH₂Cl₂ gelöst und der Reaktion hinzugefügt. Nach 1 h wird die Reaktion bei Raumtemperatur fortgeführt. Nach weiteren 48 h wird der gebildete farblose Niederschlag (N,N'-Dicyclohexylharnstoff) abfiltriert. Die klare, farblose Lösung wird nacheinander mit je drei Mal 75 ml 1 N HCl, 75 ml gesättigter NaHCO₃-Lösung sowie 75 ml gesättigter NaCl-Lösung ausgeschüttelt. Die organische Phase wird über MgSO₄ getrocknet, das Trockenmittel abfiltriert und das Lösungsmittel am Rotationsverdampfer entfernt. Man erhält 4,70 g Rohprodukt.

Die Aufreinigung erfolgt durch säulenchromatographische Trennung. Laufmittel: n-Hexan/ Ethylacetat 3:2.

Ausbeute Reinprodukt (2 Isomere: ca. 97 % (I-a4) und 3 % (I-b4)):
1,40 g farbloses Pulver (30,7 % der Theorie)

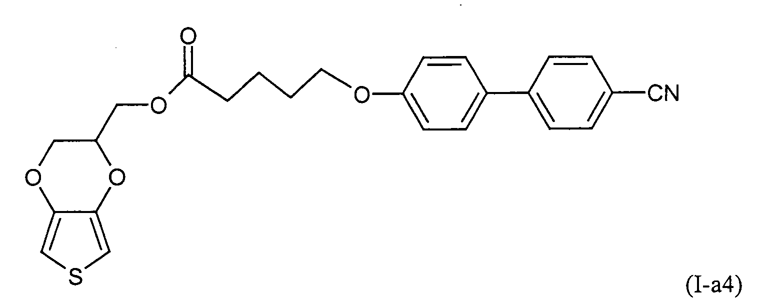

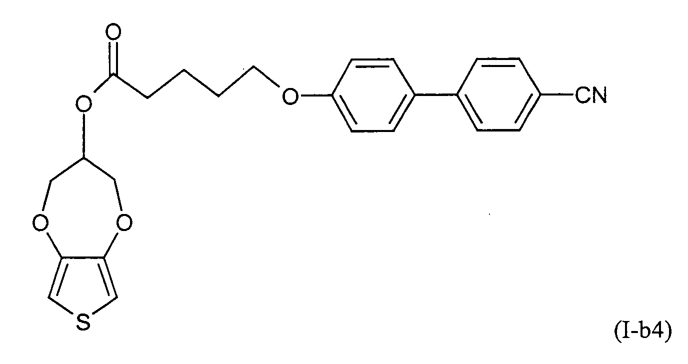

### Daten für (I-a4)/(I-b4), Stoffmengen-Verhältnis 97:3:

**IR** ν⁻¹ [cm⁻¹] = 3122, 3109; 2962, 2938, 2918, 2879, 2864; 2220; 1733; 1600, 1579, 1481; 826.
^{**1**}**H-NMR** (500 MHz, DMSO-d₆): δ [ppm] = 7,85 (d, 2H, ³*J*_{CH-CH}: 8,52 Hz); 7,81 (d, 2H, ³*J*_{CH-CH}: 8,52 Hz); 7,67 (d, 2H, ³*J*_{CH-CH}: 8,83 Hz); 7,03 (d, 2H, ³*J*_{CH-CH}: 8,83 Hz); 6,79 (s, 3 % 2H, (I-b4)); 6,59 (s, 97 % 2H); 4,44 - 4,36 (m, 1H); 4,33 - 4,20; 4,09 - 3,94 (2*m, 2*3H); 4,14 (d, 3% 4H, (I-b4)); 2,43 (t, 2H); 1,83 - 1,62 (m, 4H)
^{**13**}**C-NMR** (125,75 MHz, DMSO-d₆): δ [ppm] = 172,92; 159,62; 144,62; 141,46; 133,12; 130,67; 128,65; 127,17; 119,37; 115,44; 109,45; 100,30; 71,70; 67,56; 65,33; 62,32; 33,24; 28,25; 21,44

### Phasenübergänge (bestimmt mit dem Polarisationsmikroskop):

K 103,0 FC 105,8 1 (beim 1. Heizen, Start bei 95°C, Heizrate < 1°C/min)

| | | | |
|---|---|---|---|
| **MS** (EI, 70 eV): m/z = | 449 | M⁺ | 25,1 % |
| | 255 | C₁₂H₁₅O₄S⁺ | 68,9 % |
| | 195 | C₁₃H₉NO⁺ | 31,2 % |
| | 155 | C₇H₇O₂S⁺ | 100 % |

### Beispiel 5:

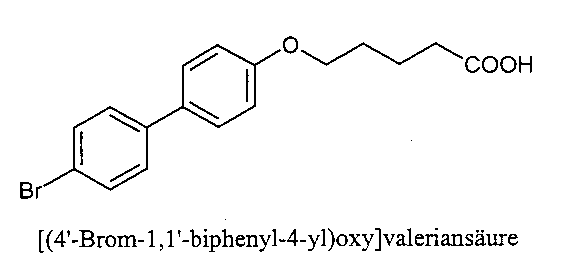

3,26 g eines Gemisches aus 80 % EDT-Methanol und 20 % Hydroxy-PDT (entsprechend 18,9 mmol; 15,1 mmol bezogen auf die Hauptkomponente EDT-Methanol), 3,14 g (15,2 mmol) N,N'-Dicyclohexylcarbodiimid und 0,3 g (2,45 mmol) 4-(Dimethylamino)-pyridin werden in 100 ml CH₂Cl₂ gelöst und in einem Eisbad abgekühlt. 5,25 g (15,0 mmol) [(4'-Brom-1,1'-biphenyl-4-yl)oxy]-valeriansäure werden spatelweise unter Rühren zu der eisgekühlten Lösung hinzugefügt. Nach 1 h wird die Reaktion bei Raumtemperatur fortgeführt. Nach weiteren 65 h wird der gebildete farblose Niederschlag (N,N'-Dicyclohexylhamstoff) abfiltriert. Die klare, farblose Lösung wird nacheinander mit je drei Mal 250 ml 1 N HCl, 150 ml gesättigter NaHCO₃-Lösung sowie einmal 150 ml und zweimal 100 ml gesättigter NaCl-Lösung ausgeschüttelt. Die organische Phase wird über MgSO₄ getrocknet, das Trockenmittel abfiltriert und das Lösungsmittel am Rotationsverdampfer entfernt. Man erhält 7,98 g Rohprodukt.

Die Aufreinigung erfolgt durch säulenchromatographische Trennung. Laufmittel: n-Hexan/ Ethylacetat 4:1.

Ausbeute (I-a5) (enthält ca. 5 % Isomer (I-b5)):
3,06 g farbloses Pulver (6,08 mmol = 40,5 % der Theorie)

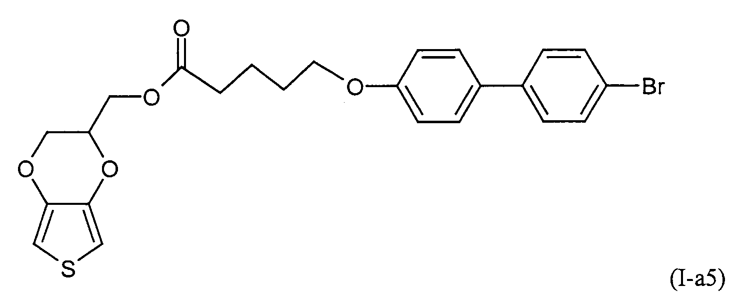

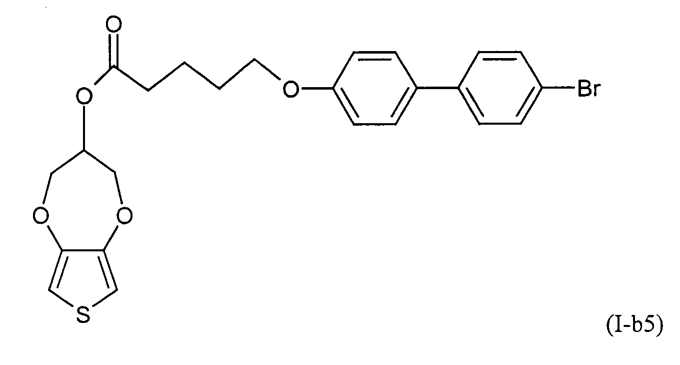

### Daten für (I-a5):

**IR** ν⁻¹ [cm⁻¹] = 3108; 2956, 2933, 2905, 2868, 1740; 1606, 1579, 1488; 815
^{**1**}**H-NMR** (500 MHz, DMSO-d₆): δ [ppm] = 7,65 - 7,50 (m, 6H); 6,99 (d, 2H); 6,79 (s, 5% 2H), 6,59 (s, 95% 2H); 4,44 - 4,36 (m, 1H); 4,33 - 4,19; 4,17 - 4,12 (m, 5% 4H); 4,07 - 3,94 (2*m, 2*3H); 2,43 (t, 2H); 1,81 - 1,63 (m, 4H)
^{**13**}**C-NMR** (125,75 MHz, DMSO-d₆): δ [ppm] = 172,92; 158,88; 141,46; 141,35; 139,37; 132,04; 131,43; 128,57; 128,05; 120,34; 115,32; 100,30; 71,70; 67,49; 65,33; 62,32; 33,25; 28,29; 21,46

### Phasenübergänge:

Am Polarisationsmikroskop wurden flüssigkristalline Phasen beobachtet.

### DSC:

*1. Heizen:* onset: 98,50°C, Maximum: 100,68°C, Endset: 103,63°C,
Heizrate: 5°C/min, 5 min bei 130°C halten

### 1. Abkühlen:

1. Peak: onset: 82,41°C, Minimum: 82,39°C, Endset: 81,58°C,
2. Peak: onset: 81,01°C, Minimum: 81,00°C, Endset: 80,75°C,
   Heizrate: - 1°C/min

### 2. Heizen:

1. Peak: onset: 82,43°C, Maximum: 84,00°C, Endset: 85,35°C,
2. Peak: onset: 92,75°C, Minimum: 94,57°C, Endset: 96,00°C,
Heizrate: 2°C/min

| | | | |
|---|---|---|---|
| **MS** (EI, 70 eV): m/z = | 502 | M⁺ | 37,4 % |
| | 255 | C₁₂H₁₅O₄S⁺ | 86,0 % |
| | 248 | C₁₂H₁₀BrO⁺ | 22,1 % |
| | 155 | C₇H₇O₂S⁺ | 100,0 % |

### Beispiel 6: Neutrales Polythiophen hergestellt aus (I-a5) und (I-b5)

0,3 g (0,6 mmol) des gemäß Beispiel 5 hergestellten Thiophengemisches (I-a5/I-b5, Stoffmengen-Verhältnis 95 : 5) werden in 12 ml Chloroform gelöst. In dieser Lösung werden 0,15 g (1,5 mmol) fein gepulvertes Calciumcarbonat aufgeschlämmt. Zu diesem Gemisch werden bei 23°C unter Rühren im Abstand von 1 h in zwei gleichen Portionen 0,24 g (1,48 mmol) Eisen-III-chlorid (wasserfrei) gegeben. Das Gemisch wird weitere 4 h bei 23°C gerührt. Danach wird mit 12 ml Methylenchlorid. Nach Zugabe von 6 ml wässrigem Ammoniak (26 %ig) wird 2 h zum Rückfluss erhitzt. Anschließend wird vom Feststoff abfiltriert. Der Feststoff wird mit Chloroform gewaschen und die vereinigten organischen Phasen erneut mit 6 ml wässrigem 26 %igem Ammoniak unter intensivem Rühren für 1 h zum Rückfluss erhitzt. Die organische Phase wird mit 0,05-molarer EDTA-Lösung (Na-Ethylendiamintetraacetat) und anschließend mit Wasser gewaschen, über Natriumsulfat getrocknet und eingeengt. Der feste Rückstand wird in Methanol für 30 min aufgekocht und heiß abfiltriert. Man erhält 0,13 g (43,3 % d. Th.) Polythiophen mit wiederkehrenden Einheiten (IV-a5) und (IV-b5).

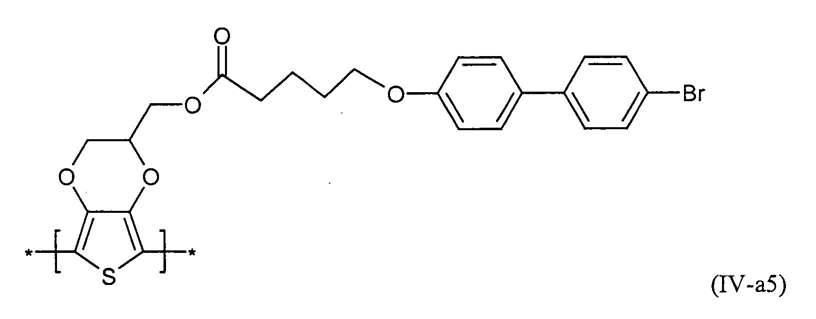

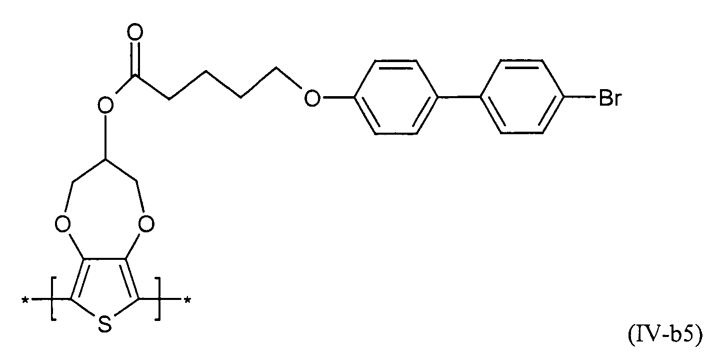

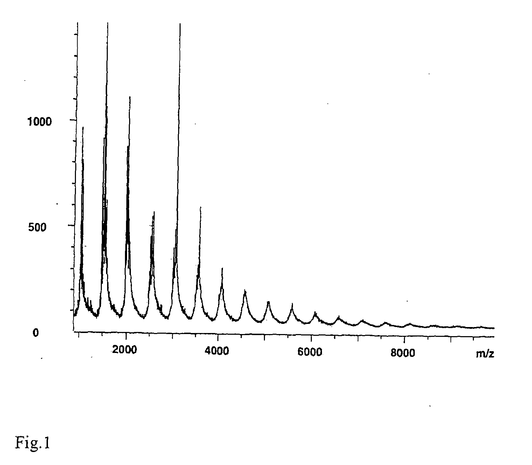

### Charakterisierung durch MALDI-TOF-MS (Fig. 1).

### Beispiel 7: Neutrales Polythiophen hergestellt aus (I-a5) und (I-b5)

0,6 g (1,19 mmol) des gemäß Beispiel 5 hergestellten Thiophens (I-a5/I-b5, Stoffmengen-Verhältnis 95 : 5) werden in 24 ml Chloroform gelöst. In dieser Lösung werden 0,475 g (4,75 mmol) fein gepulvertes Calciumcarbonat aufgeschlämmt. Zu diesem Gemisch werden bei 23°C unter Rühren im Abstand von jeweils 1 h in drei gleichen Portionen 0,77 g (4,75 mmol) Eisen-III-chlorid (wasserfrei) gegeben. Das Gemisch wird weitere 4 h bei 23°C gerührt. Danach wird mit 24 ml Methylenchlorid. Nach Zugabe von 12 ml wässrigem Ammoniak (26 %ig) wird 2 h zum Rückfluss erhitzt. Anschließend wird vom Feststoff abfiltriert. Der Feststoff wird mit Chloroform gewaschen und die vereinigten organischen Phasen erneut mit 12 ml wässrigem 26 %igem Ammoniak unter intensivem Rühren für 1 h zum Rückfluss erhitzt. Die organische Phase wird mit 0,05-molarer EDTA-Lösung (Na-Ethylendiamintetraacetat) und anschließend mit Wasser gewaschen, über Natriumsulfat getrocknet und eingeengt. Der feste Rückstand wird in Methanol für 30 min aufgekocht und heiß abfiltriert. Man erhält 0,2 g (33,3 % d. Th.) Polythiophen mit wiederkehrenden Einheiten (IV-a5) und (IV-b5).

### Charakterisierung durch Gel-Permeations-Chromatographie (in THF, Polystyrol-Standard, RI-Detektion): Mₙ = 2300; M_{w} = 9900

### Beispiel 8: Kationisches, hochleitfähiges Polythiophen aus (I-a5) und (I-b5) als Beschichtung durch in situ-Polymerisation

0,22 g des gemäß Beispiel 5 hergestellten Thiophens (I-a5/I-b5, Stoffmengen-Verhältnis 95 : 5) werden in 6,625 g siedendem n-Butanol gelöst. Zu der heißen Lösung werden rasch 1,562 g einer 40 %igen Lösung von Eisen-III-tosylat in n-Butanol (Baytron® CB 40; Hersteller: H. C. Starck GmbH) gegeben und das Gemisch auf eine auf ca. 100°C vorgewärmte Glasplatte aufgerakelt (Nassfilm-Schichtdicke 24 µm). Nach 10 min Trocknung bei 100°C und Abkühlen auf Raumtemperatur (23°C) wird die Beschichtung mit entionisiertem Wasser gewaschen und föngetrocknet. Als Oberflächenwiderstand (Zweipunktmethode) werden 461 Ω/square gemessen. Die Platte wurde nochmals 30 min bei 150° getempert, als Oberflächenwiderstand nach Abkühlen auf 23°C wurden 211 Ω/square gemessen.

### Beispiel 9: Kationisches Polythiophen (Tetrachloroferrat) aus (I-a5) und (I-b5) als Pulver

Unter N₂-Schutzgas werden 1,0 g (1,99 mmol) des gemäß Beispiel 5 hergestellten Thiophens (I-a5/I-b5, Stoffmengen-Verhältnis 95 : 5) in 7,7 ml Acetonitril gelöst und zum Rückfluss erhitzt. Zu dieser Lösung werden innerhalb von 30 min unter Rückfluss 1,875 g (11,56 mmol) Eisen-III-chlorid (wasserfrei) in 19 ml Acetonitril getropft. Die Lösung wird weitere 16 h unter Rückfluss erhitzt. Anschließend wird der unlösliche Niederschlag an kationischem Polythiophen abgesaugt und mehrmals mit Acetonitril gewaschen. Ausbeute 0,89 g kationisches Polythiophen mit Tetrachloroferrat als Gegenanion.

### Beispiel 10:

### a) Umsetzung eines EDT-Methanol/Hydroxy-PDT Gemisches mit 1,6-Dibromhexan (Reaktand enthaltend die Gruppierung B):

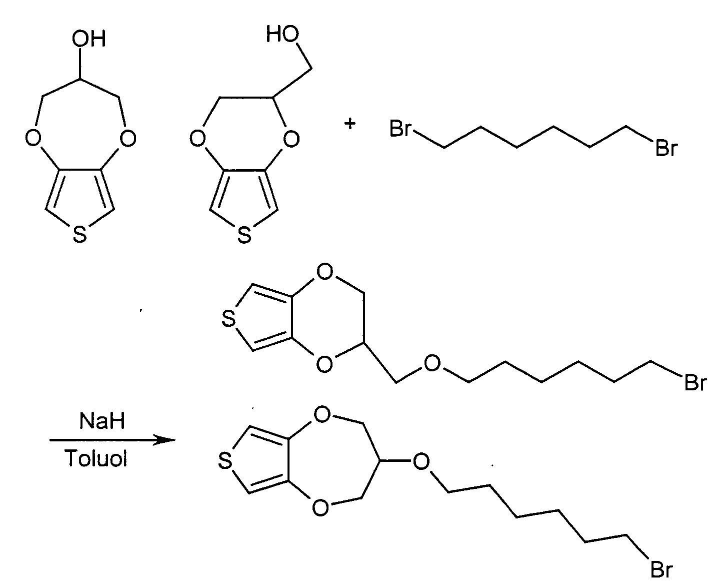

Es wird unter Ar-Schutzgasatmosphäre gearbeitet. 3,03 g Natriumhydrid (60 % in Mineralöl, 75,8 mmol) werden zweimal mit trockenem Toluol gewaschen und danach in 100 ml Toluol suspendiert. Die Suspension wird auf 80°C geheizt und 10 g EDT-Methanol/Hydroxy-PDT (80:20, 58,1 mmol (bezogen auf das Gemisch)) gelöst in 80 ml Toluol langsam innerhalb von 40 min zugetropft. Die farblose Suspension wird dabei gelblich braun. Nach einer weiteren Stunde werden 70,9 g (290,5 mmol) 1,6-Dibromhexan hinzugefügt. Die Lösung wird 100 min bei 80°C gerührt, dann wird die Heizung ausgestellt und bei Raumtemperatur (23°C) weitere 20 h gerührt. Es werden 15 ml Aceton hinzugegeben und nach einer halben Stunde werden noch 8 ml Wasser hinzugefügt. Dabei löst sich der farblose Niederschlag auf und es entstehen 2 Phasen. Die Reaktionsmischung wird zweimal mit je 90 ml 1 molarer (M) HCl ausgeschüttelt. Die Toluol-Phase wird noch mal mit 180 ml 1 M HCl extrahiert.

Die gesammelten HCl-Phasen werden dreimal mit 90 ml Chloroform extrahiert. Die gesammelten Chloroform-Phasen werden verworfen und die HCl-Phase dreimal mit 50 ml Toluol extrahiert. Die HCl-Phase wird danach verworfen. Die gesammelten Toluol-Phasen werden dreimal mit je 120 ml gesättigter wässriger NaCl-Lösung ausgeschüttelt. Die Toluol-Phase wird über MgSO₄ getrocknet, das Trockenmittel abfiltriert und das Lösungsmittel am Rotationsverdampfer entfernt. Die so erhaltene braune Flüssigkeit wird durch Säulenchromatographie gereinigt (Ethylacetat:Hexan zunächst 1:19 später 1:4).

**Ausbeute:** 12,95 g (38,6 mmol (bezogen auf das Gemisch) = 66,5 % der Theorie) gelbliche Kristalle, Gehalt an PDT-Derivat: 16 %.

### b) Umsetzung des Produktgemisches aus a) mit 4-(4-Bromphenyl)phenol (Reaktand enthaltend die Gruppierung M)

1,13 g (4,54 mmol) 4-(4-Bromphenyl)phenol, 1,69 g (5,04 mmol) des Produktgemisches aus a) (16 % PDT-Derivat, 84 % EDT-Derivat und 1,81 g (5,56 mmol) Cs₂CO₃ werden in 30 ml DMF suspendiert und 70 h bei Raumtemperatur gerührt. Zu der Suspension werden 90 ml Puffer pH 7 (Aldrich) gegeben und die Lösung wird zweimal mit je 100 ml Diethylether extrahiert. Die gesammelten Ether-Phasen werden über MgSO₄ getrocknet, das Trockenmittel abfiltriert und das Lösungsmittel am Rotationsverdampfer entfernt. Das Rohprodukt wird aus Methanol/Methylenchlorid umkristallisiert. Die erhaltenen farblosen Kristalle werden abfiltriert, mit Methanol gewaschen und im Vakuum über P₂O₅ getrocknet.
- **Ausbeute:**: 1.Fraktion: 1,57 g (3,12 mmol = 68,7 % der Theorie) farblose Kristalle (I-a6) im Gemisch mit 14 % (I-b6).
2.Fraktion: 0,33 g (0,66 mmol = 14,4 % der Theorie) farblose, blättchenartige Kristalle (I-a6) im Gemisch mit 15 Mol-% (I-b6).

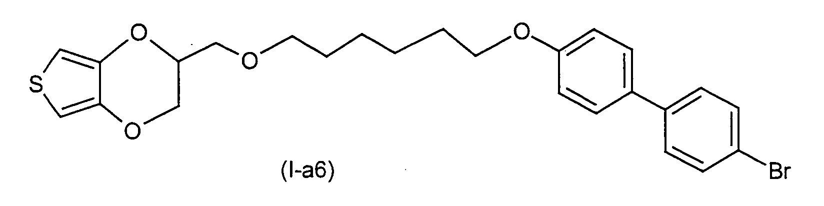

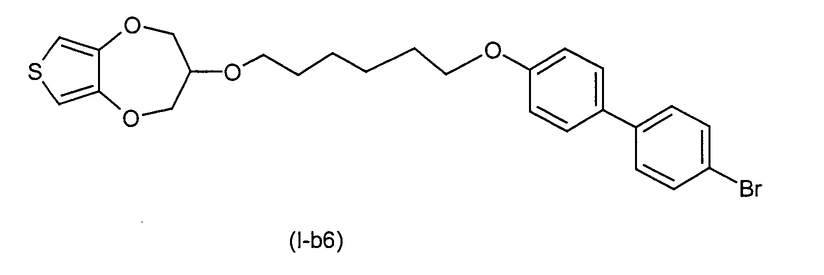

**IR** ν⁻¹ [cm⁻¹] = 2934, 2868, 2859,1604, 1580,1491, 806.
^{**1**}**H-NMR** (500 MHz, CDCl₃): δ [ppm] = 7,52 (d, 2H, ³*J*_{CH-CH}: 8,51 Hz); 7,47 (d, 2H, ³*J*_{CH-CH}: 8,83 Hz); 7,41 (d, 2H, ³*J*_{CH-CH}: 8,51 Hz); 6,95 (d, 2H, ³*J*_{CH-CH}: 8,83 Hz); 6,46 (s, 14 % 2H); 6,33, 6,32 (2*d, je 86 % 1H); 4,34-4,27 (m, 86 % 1H); 4,27-4,20 (m, 86 % 1H); 4,10 (dd, 14 % 4H); 4,05 (dd, 86 % 1H); 3,99 (t, 2H); 3,87-3,80 (m, 14 % 1H); 3,69 (dd, 86 % 1H, *J*_{CH-CH}: 10,40 Hz); 3,60 (dd, 86 % 1H, *J*_{CH-CH}: 10,40 Hz); 3,56 (t, 14 % 1H); 3,51 (t, 86 % 2H); 1,81 (m, 2H); 1,63 (m, 2H); 1,54-1,39 (2*m, je 2H)
^{**13**}**C-NMR** (125,75 MHz, DMSO-d⁶): δ [ppm] = 158,97; 149,42; 141,73, 141,67; 139,37; 132,03; 131,34; 128,54, 128,04; 120,32; 115,30; 105,92; 100,04, 99,97; 77,34, 71,61, 68,91; 72,82, 71,16, 68,87, 67,82, 65,85; 29,71; 29,35, 28,98; 25,69.

### Phasenübergänge (bestimmt mit dem Polarisationsmikroskop):

K 86,2 FC 87,7 1 (beim 1. Heizen)
K 86,2 FC 88,1 I (beim 2. Heizen)

| | | | |
|---|---|---|---|
| **MS** (E1, 70 eV): m/z = | 502 | M⁺ | 96,6 % |
| | 248 | C₁₂H₉BrO⁺ | 63,5 % |
| | 172 | C₇H₈O₃S⁺ | 19,8 % |
| | 83 | C₄H₃S⁺ | 64,6 % |

### Beispiel 11:

### a) Herstellung eines Reaktanden enthaltend die Gruppierung M

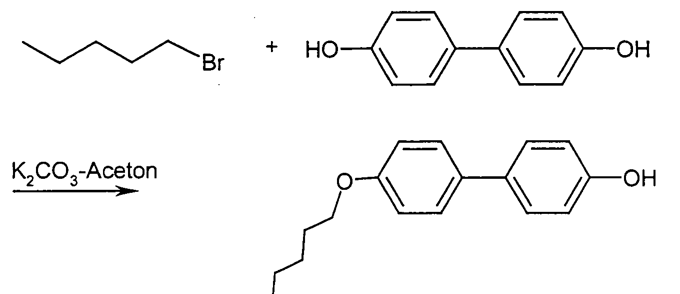

3,22 g (17,3 mmol) 4,4'-Dihydroxybiphenyl, 2,62 g (17,3 mmol) 1-Brompentan und 2,53 g (18,3 mmol) K₂CO₃ werden in 150 ml Aceton suspendiert und 26 h unter Rückfluss gekocht, danach wird weitere 24 h bei Raumtemperatur gerührt. Der Niederschlag wird abfiltriert, verworfen und dem Filtrat wird am Rotationsverdampfer das Aceton komplett entzogen. Der entstandene gelbliche Niederschlag wird durch eine säulenchromatographische Trennung aufgereinigt (4 Hexan : 1 Ethylacetat).
- **Ausbeute:**: 2,07 g (8,08 mmol = 46,7 % der Theorie) cremefarbene Kristalle.

### b) Umsetzung des Produktgemisches aus Beispiel 10a) mit dem Reaktanden aus Beispiel 11a)

0,50 g (1,95 mmol) des Reaktanden aus Beispiel 11a), 0,75 g (2,24 mmol) des Produktgemisches aus Beispiel 10a) (16 % PDT-Derivat, 84 % EDT-Derivat) und 0,76 g (2,33 mmol) Cs₂CO₃ werden in 15 ml DMF suspendiert und 5 Tage bei Raumtemperatur gerührt. Zu der Suspension werden 50 ml gesättigte wässrige NaHCO₃-Lösun gegeben und es wird zweimal mit je 50 ml Chloroform extrahiert. Die gesammelten Chloroform-Phasen werden nochmals mit 50 ml gesättigter wässriger NaHCO₃-Lösun ausgeschüttelt; danach wird die Chloroform-Phase je zweimal mit 50 ml gesättigter wässriger NaCl-Lösung ausgeschüttelt. Die Chloroform-Phase wird über MgSO₄ getrocknet, das Trockenmittel abfiltriert und das Lösungsmittel am Rotationsverdampfer entfernt. Das Rohprodukt wird aus Methanol/Methylenchlorid umkristallisiert. Die erhaltenen beigen Kristalle werden abfiltriert, mit Methanol gewaschen und im Vakuum über P₂O₅ getrocknet.
- **Ausbeute:**: 0,87 g (1,7 mmol = 87,4 % der Theorie (bezogen auf das Gemisch)) (I-a7) im Gemisch mit 15,5% (I-b7)

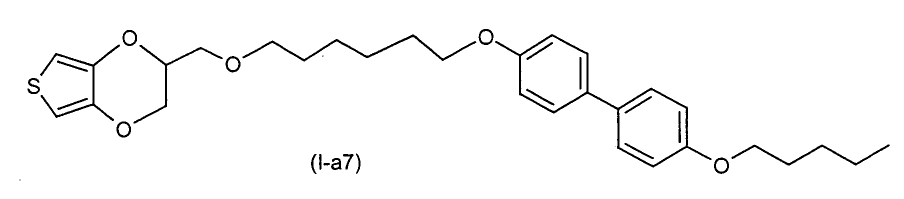

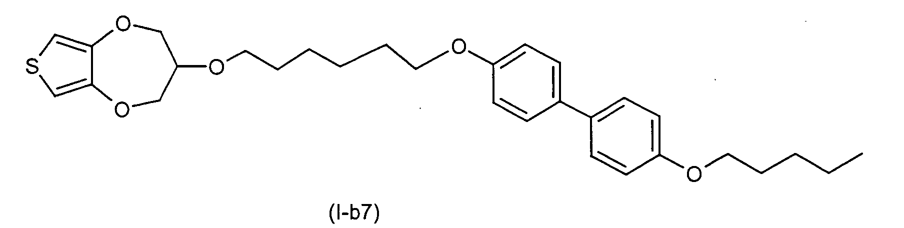

**IR** ν⁻¹ [cm⁻¹] = 3040, 3067, 2957, 2936, 2861, 1606, 1568, 1491, 807.
^{**1**}**H-NMR** (500 MHz, CDCl₃): δ [ppm] = 7,51-7,41 (m, 4H); 6,99-6,89 (m, 4H); 6,46 (s, 15,5 % 2H); 6,33, 6,32 (2*d, 84,5 % 2H); 4,33-4,27 (m, 84,5 % 1H); 4,26-4,20 (m, 84,5 % 1H); 4,09 (dd, 15,5 % 4H); 4,05 (dd, 84,5 % 1H); 3,98 (t, 4H, 13-H); 3,86-3,80 (m, 15,5 % 1H); 3,68 (dd, 84,5 % 2H, *J*_{CH-CH}: 10,40 Hz); 3,59 (dd, 84,5 % 2H, *J*_{CH-CH}: 10,40 Hz); 3,55 (t, 15,5 % 1H); 3,51 (t, 84,5 % 2H); 1,86-1,74 (m, 4H); 1,63 (m, 2H); 1,55-1,33 (m, 8H); 0,94 (t, 3H).
^{**13**}**C-NMR** (125,75 MHz, CDCl₃: δ [ppm] = 157,20, 157,12; 148,11; 140,57, 140,53; 132,35 132,25; 126,62; 113,69; 104,25; 98,65, 98,53; 76,83, 70,64; 71,62, 70,87, 68,10, 67,03, 66,85, 65,19; 28,80; 28,45, 28,22, 27,98, 27,20; 24,87, 24,81; 21,46; 13,03.

### Phasenübergänge (bestimmt mit dem Polarisationsmikroskop):

K 101,1 FC 103,1 I (beim 1. Heizen, Start 92°C, < 1°C/min)

| | | | |
|---|---|---|---|
| **MS** (EI, 70 eV): m/z = | 510 | M⁺ | 100,0 % |
| | 256 | C₁₇H₂₀O₂⁺ | 10,8 % |
| | 186 | C₁₂H₁₀O₂⁺ | 51,9 % |
| | 83 | C₄H₃S⁺ | 15,3 % |

### Beispiel 12:

### a) Umsetzung eines EDT-Methanol-Hydroxy-PDT Gemisches mit 1,5-Dibrompentan (Reaktand enthaltend die Gruppierung B):

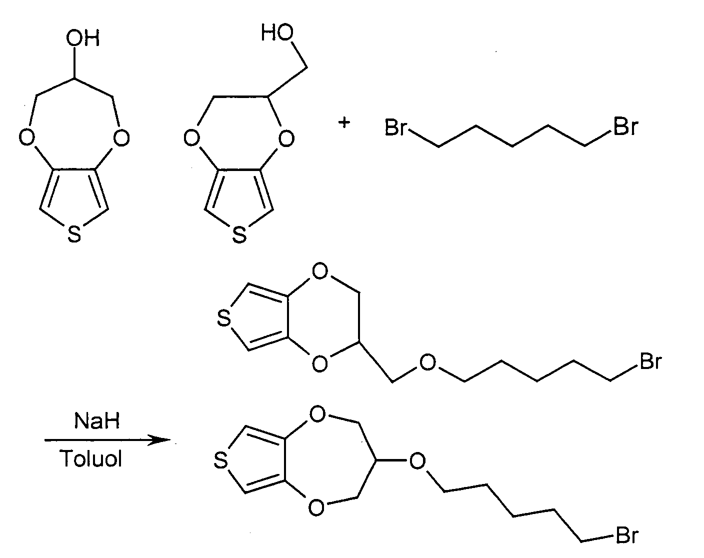

Es wird unter Stickstoff gearbeitet. 8 g Natriumhydrid (60 % in Mineralöl, 0,2 mol) werden zweimal mit trockenem Toluol gewaschen und danach in 200 ml Toluol suspendiert. Die Suspension wird auf 80°C geheizt und 20 g EDT-Methanol/Hydroxy-PDT (80:20, 116,2 mmol, bezogen auf das Gemisch) gelöst in 160 ml Toluol innerhalb von 1,5 h zugetropft. Die farblose Suspension wird dabei braun. Nach einer weiteren Stunde werden 134 g (581 mmol) 1,5-Dibrompentan hinzugefügt. Die Lösung wird 2 h bei 80°C gerührt, dann wird die Heizung ausgestellt und bei Raumtemperatur weitere 12 h gerührt. 16 ml Wasser wird zum Abbruch der Reaktion vorsichtig hinzugefügt. Die Reaktionsmischung wird zwei Mal mit je 180 ml 1 N HCl ausgeschüttelt. Die Toluol-Phase wird noch einmal mit 360 ml 1 N HCl ausgeschüttelt. Die gesammelten HCl-Phasen werden dreimal mit 180 ml Chloroform extrahiert. Die gesammelten Chloroform-Phasen werden verworfen und die HCl-Phase dreimal mit 100 ml Toluol extrahiert. Die HCl-Phase wird danach verworfen. Die gesammelten Toluol-Phasen werden dreimal mit je 240 ml gesättigter wässriger NaCl-Lösung ausgeschüttelt. Die Toluol-Phase wird über MgSO₄ getrocknet, das Trockenmittel abfiltriert und das Lösungsmittel am Rotationsverdampfer entfernt. Das überschüssige 1,5-Dibrompentan wird im Hochvakuum bei 60°C abdestilliert. Das Rohprodukt wird säulen-chromatographisch gereinigt (Ethylacetat : Hexan 1:4).
**Ausbeute**: 19,49 g (60,7 mmol = 52,1 % d. Theorie) grünliches Öl, Gehalt an PDT-Derivat: 7 %

### b) Umsetzung des Produktgemisches aus a) mit 4-(4-Bromphenyl)phenol (Reaktand enthaltend die Gruppierung M)

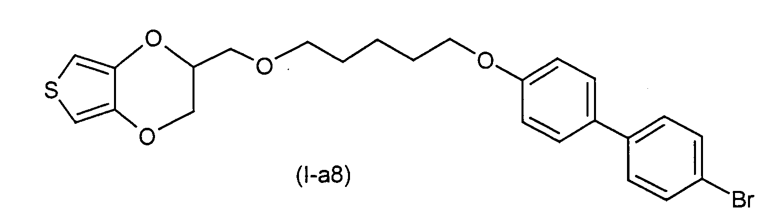

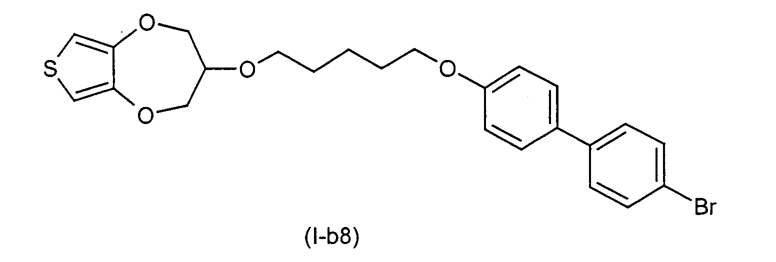

1,43 g (5,74 mmol) 4-(4-Bromphenyl)phenol, 1,93 g (6,01 mmol) des Produktgemisches aus a) (7 % PDT-Derivat, 93 % EDT-Derivat) und 2,56 g (7,86 mmol) Cs₂CO₃ werden in 50 ml DMF suspendiert und 4 d bei Raumtemperatur gerührt. Die Aufarbeitung erfolgt analog Beispiel 10 b).

Das Rohprodukt wird aus Methanol/Methylenchlorid umkristallisiert. Der farblose Niederschlag wird abfiltriert, mit Methanol gewaschen und im Vakuum getrocknet.
- **Ausbeute**:: 1. Fraktion: 1,87 g (3,82 mmol = 66,6 % der Theorie) (I-a8) im Gemisch mit 5 % (I-b8)
2. Fraktion: 0,57 g (1,17 mmol = 20,3 % der Theorie) (I-a8) im Gemisch mit 9 % (I-b8)
3. Fraktion 0,17 g (0,35 mmol = 6,1 % der Theorie) (I-a8) im Gemisch mit 31 % (I-b8).

**IR** ν⁻¹ [cm⁻¹] = 2942, 2912, 2870 1604, 1579, 1482 816.
^{**1**}**H-NMR** (500 MHz, CDCl₃): δ [ppm] = 7,52, 7,40 (2*d, je 2H); 7,46 (d, 2H); 6,95 (d, 2-H); 6,46 (s, 5 % 2H); 6,33, 6,32 (2*d, je 95 % 1H); 4,36 - 4,27 (m, 95 % 1H); 4,24 (dd, 95 % 1H, *J*_{CH-CH}: 11,67 Hz, überlagert dd); 4,10 (dd, 5 % 2H); 4,06 (dd, 95 % 1H, *J*_{CH-CH}: 11,67 Hz); 3,99 (t, 2H); 3,88 - 3,80 (m, 5 % 1H); 3,69 (dd, 95 % 1H, *J*_{CH-CH}: 10,40 Hz); 3,60 (dd, 95 % 1H, *J*_{CH-CH}: 10,40 Hz); 3,53 (t, 95 % 2H); 1,83, 1,67 (2*m, je 2H); 1,62 - 1,47 (m, 2H)
^{**13**}**C-NMR** (125,75 MHz, CDCl₃): δ [ppm] = 159,28; 141,98, 141,94; 140,16; 132,73; 132,17; 128,67, 128,35; 121,13; 115,27; 100,10, 99,98; 73,04, 72,20; 69,58, 68,25, 66,61; 29,69, 29,44; 23,08

**Phasenübergänge** (bestimmt mit dem Polarisationsmikroskop):
K 109,1°C FC ca. 120°C I (1. Heizen)
K 107,7°C FC ca. 126°C I (2. Heizen)

| **Elementaranalyse:** | | |
|---|---|---|
| berechnet | 58,90 % C | 5,15 % H |
| gefunden | 59,11 % C | 5,11 % H |

| | | | |
|---|---|---|---|
| **MS** (EI, 70 eV): m/z = | 488 | M⁺ | 38,9 % |
| | 241 | C₁₂H₁₇O₃S⁺ | 54,7 % |
| | 155 | C₇H₇O₂S⁺ | 38,6 % |

### Beispiel 13: Kationisches, hochleitfähiges Polythiophen aus (I-a8) und (I-b8) als Beschichtung durch in situ-Polymerisation

0,21 g des gemäß Beispiel 12 hergestellten Thiophens (I-a8)/(I-b8) (Stoffmengen-Verhältnis 95 : 5) werden in 6,625 g n-Butanol heiß gelöst. Zu der heißen Lösung werden rasch 1,562 g einer 40 %igen Lösung von Eisen-III-tosylat in n-Butanol (Baytron® CB 40; Hersteller: H. C. Starck GmbH) gegeben und das Gemisch auf eine auf ca. 80°C vorgewärmte Glasplatte aufgerakelt (Nassfilm-Schichtdicke 24 µm). Nach 10 min Trocknung bei 80°C und Abkühlen auf Raumtemperatur (23°C) wird die Beschichtung mit entionisiertem Wasser gewaschen und föngetrocknet. Als Oberflächenwiderstand (Zweipunktmethode) werden 601 Ω/square gemessen. Die Platte wird nochmals 30 min bei 150° getempert, als Oberflächenwiderstand nach Abkühlen auf 23°C werden 190 Ω/square gemessen.

### Beispiel 14:

### a) Umsetzung eines EDT-Methanol/Hydroxy-PDT Gemisches mit 1,6-Dibromhexan (Reaktand enthaltend die Gruppierung B):

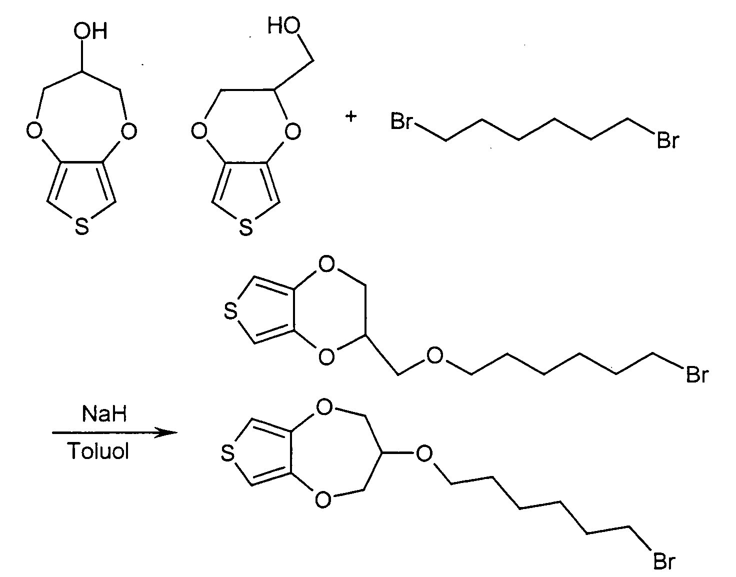

Die Umsetzung wird entsprechend Beispiel 10 a) durchgeführt.

### b) Umsetzung des Produktgemisches aus a) mit 4-(4-Propylphenyl)benzoesäure (Reaktand enthaltend die Gruppierung M)

1,00 g (4,16 mmol) 4-(4-Propylphenyl)benzoesäure, 1,57 g (4,68 mmol) des Produktgemisches aus a) (16 % PDT-Derivat, 84 % EDT-Derivat) und 1,67 g (5,13 mmol) Cs₂CO₃ werden in 46 ml DMF suspendiert und 5 d bei Raumtemperatur gerührt. Zu der Suspension werden 50 ml gesättigte wässrige NaHCO₃-Lösung gegeben, die Lösung wird zwei Mal mit je 50 ml Chloroform extrahiert. Zur besseren Phasentrennung wird 50 ml Wasser hinzugegeben. Die gesammelten organischen Phasen werden mit 75 ml wässr. NaHCO₃-Lsg, erneut ausgeschüttelt und danach zweimal mit je 50 ml gesättigter wässriger NaCl-Lösung ausgeschüttelt. Die Chloroform-Phase wird über MgSO₄ getrocknet, das Trockenmittel abfiltriert und das Lösungsmittel am Rotationsverdampfer und im Ölpumpenvakuum entfernt.

Das beige Rohprodukt wird säulenchromatographisch gereinigt (Laufmittel Hexan/Essigester 4 : 1). Man erhält eine klare Flüssigkeit, die im Gefrierschrank zu einem farblosen Niederschlag kristallisiert. 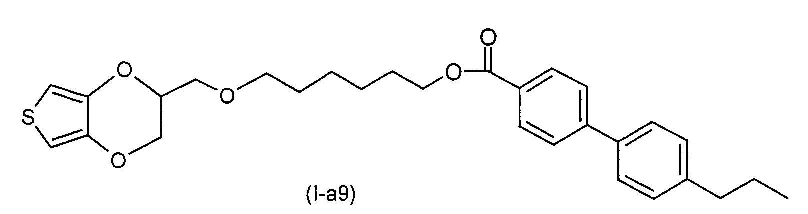 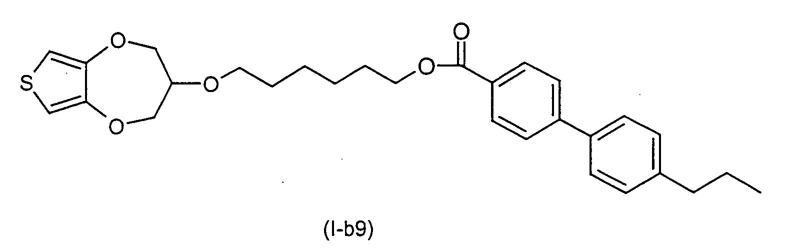
- **Ausbeute**:: 2,00 g (4,04 mmol = 97 % d. Theorie) (I-a9) im Gemisch mit 15 % (I-b9)
**IR** ν⁻¹ [cm⁻¹] = 2931, 2866; 1711; 1607, 1580, 1485; 835
^{**1**}**H-NMR** (500 MHz, CDCl₃): δ [ppm] = 8,09 (d, 2H); 7,65 (d, 2H); 7,54, 7,28 (2*d, je 2H); 6,46 (s, 15 % 2H); 6,33, 6,31 (2*d, je 85 % 1H, ⁴*J*_{CH-CH}: 3,47 Hz); 4,34 (t, 2H); 4,32 - 4,26 (m, teilweise überlagert 85 % 1H); 4,23 (dd, 85 % 1H überlagert dd); 4,09 (dd, überlagert; 15 % 2H); 4,05 (dd, 85 % 1H); 3,87 - 3,80 (m, 15 % 1H); 3,68 (dd, 85 % 1H, *J*_{CH-CH}: 10,40 Hz); 3,59 (dd, 85 % 1H, *J*_{CH-CH}: 10,40 Hz); 3,55 (t, 85 % 1H); 3,51 (t, 85 % 2H); 2,64 (t, 2H); 1,85 - 1,74 (m, 2H); 1,74 * 1,56 (m, 4H); 1,54 - 1,37 (m, 4H); 0,97 (t, 3H)
^{**13**}**C-NMR** (125,75 MHz, CDCl₃): δ [ppm] = 166,60; 145,53, 142,60, 137,34; 141,57, 141,54; 130,02, 129,06, 127,08, 126,79; 128,87; 99,67, 99,55; 77,88; 72,53, 71,87; 71,65, 69,82; 69,14, 66,21, 64,91; 37,70; 29,80; 29,44, 28,72; 25,90, 25,76; 24,52; 13,86

**Phasenübergänge** (bestimmt mit dem Polarisationsmikroskop):
K 49,1 °C FC 56,4°C I (1. Heizen, < 1°C/min)
K 53,1 °C FC 58,1 °C I (2. Heizen, < 1°C/min)

**Phasenübergänge** (bestimmt mit DSC):
*1. Heizen*: onset: 50,58°C, Maximum: 53,19°C, Endset: 57,38°C,
*2. Heizen*: onset: 46,88°C, Maximum: 49,35°C, Endset: 52,77°C,
*3. Heizen*: onset: 45,25°C, Maximum: 49,44°C, Endset: 52,82°C,
   Heizrate stets 1°C/min

| **Elementaranalyse:** | | |
|---|---|---|
| berechnet | 70,42 % C | 6,93 % H |
| gefunden | 70,49 % C | 6,91 % H |

| | | | |
|---|---|---|---|
| **MS** (EI, 70 eV): m/z = | 494 | M⁺ | 7,6 % |
| | 255 | C₁₃H₁₀O₃S⁺ | 11,2 % |
| | 223 | C₁₆H₁₅O⁺ | 24,3 % |
| | 83 | C₄H₃S⁺ | 32,4 % |

### Beisniel 15: Kationisches, hochleitfähiges Polythiophen aus (I-a9) und (I-b9) als Beschichtung durch in situ-Polymerisation

0,215 g des gemäß Beispiel 14 hergestellten Thiophens (I-a9)/(I-b9) (Stoffmengen-Verhältnis 85 : 15) werden in 6,625 g n-Butanol warm gelöst. Zu der Lösung werden rasch 1,562 g einer 40 %igen Lösung von Eisen-III-tosylat in n-Butanol (Baytron® CB 40; Hersteller: H. C. Starck GmbH) gegeben und das Gemisch auf eine auf ca. 80°C vorgewärmte Glasplatte aufgerakelt (Nassfilm-Schichtdicke 24 µm). Nach 10 min Trocknung bei 80°C und Abkühlen auf Raumtemperatur (23°C) wird die Beschichtung mit entionisiertem Wasser gewaschen und föngetrocknet. Als Oberflächenwiderstand (Zweipunktmethode) werden 473 Ω/square gemessen. Die Platte wird nochmals 30 min bei 150°C getempert, als Oberflächenwiderstand nach Abkühlen auf 23°C werden 110 Ω/square gemessen.

### Beispiel 16:

### a) Umsetzung eines EDT-Methanol/Hydroxy-PDT Gemisches mit 1,4-Dibrombutan (Reaktand enthaltend die Gruppierung B):

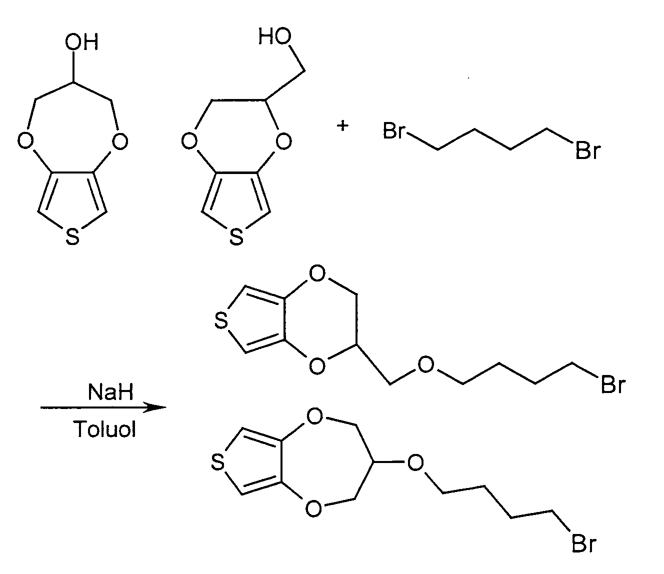

Es wird unter Stickstoff gearbeitet. 4 g Natriumhydrid (60 % in Mineralöl, 0,1 mol) werden zwei Mal mit trockenem Toluol gewaschen, danach in 100 ml Toluol suspendiert. Diese Suspension wird auf 80°C geheizt und 10 g EDT-Methanol/Hydroxy-PDT (ca. 80:20, 58,1 mmol, bezogen auf das Gemisch) gelöst in 80 ml Toluol innerhalb von 35 min zugetropft. Die farblose Suspension wird dabei braun. Nach einer weiteren Stunde werden 45 ml (82,35 g = 0,381 mol) 1,4-Dibrombutan hinzugefügt. Die Lösung wird 5 h bei 80°C gerührt, dann wird die Heizung abgestellt und bei Raumtemperatur weitere 12 h gerührt. 8 ml Wasser wird zum Abbruch der Reaktion vorsichtig hinzugefügt. Die Reaktionsmischung wird zwei Mal mit je 90 ml 1 N HCl ausgeschüttelt. Die Toluol-Phase wird noch einmal mit 180 ml 1N HCl ausgeschüttelt. Die gesammelten HCl-Phasen werden dreimal mit 90 ml Chloroform extrahiert. Die gesammelten Chloroform-Phasen werden verworfen und die HCl-Phase dreimal mit 50 ml Toluol extrahiert. Die HCl-Phase wird danach verworfen. Die gesammelten Toluol-Phasen werden dreimal mit je 120 ml gesättigter wässriger NaCl-Lösung ausgeschüttelt. Die Toluol-Phase wird über MgSO₄ getrocknet, das Trockenmittel abfiltriert und das Lösungsmittel am Rotationsverdampfer entfernt. Das überschüssige 1,4-Dibrombutan wird im Ölpumpenvakuum bei 60°C abdestilliert. Das Rohprodukt wird säulenchromato-graphisch gereinigt (Laufmittel Ethylacetat/Hexan 1 : 4).
- **Ausbeute:**: 8,15 g (26,5 mmol = 45,7 % d. Theorie) grünlich-braune Flüssigkeit mit einem Gehalt an PDT-Derivat von 12 %
**IR**ν⁻¹ [cm⁻¹] = 3110 2938, 2918, 2868;, 1482, 1452, 1427, 1374, 1247, 1184, 1123, 1019,856,758
^{**1**}**H-NMR** (500 MHz, CDCl₃): δ [ppm] = 6,47 (s, 12% 2H); 6,34, 6,33 (2*d, je 88% 1H, ⁴*J*_{CH-CH}: 3,78 Hz); 4,37 - 4,27 (m, 93 % 1H); 4,23 (dd, 88% 1H, *J*_{CH-CH}: 11,66 Hz, mit Überlagerung); 4,10 (dd, 12% 4H); 4,05 (dd, 1H, *J*_{CH-CH}: 11,66 Hz); 3,88 - 3,80 (m, 12% 1H); 3,69 (dd, 88% 1H, *J*_{CH-CH}: 10,40 Hz); 3,60 (dd, 88% 1H, *J*_{CH-CH}: 10,40 Hz); 3,59 (t, 7 % 1H); 3,54 (t, 88% 2H); 3,44 (t, 2H); 2,07 - 1,64 (2*m, je 2H)
^{**13**}**C-NMR** (125,75 MHz, CDCl₃): δ [ppm] = 149,46; 141,92, 141,86; 105,74; 100,11, 99,03; 78,34; 71,92, 69,15; 72,99, 71,30, 69,58, 66,52; 34,00; 29,89, 28,51; 28,88

| **Elementaranalyse:** | | |
|---|---|---|
| berechnet | 43,01 % C | 4,92 % H |
| gefunden | 43,11 % C | 4,88 % H |

| | | | |
|---|---|---|---|
| **MS** (EI, 70 eV): m/z = | 306 | M⁺ | 27,6 % |
| | 227 | C₁₁H₁₅O₃S⁺ | 25,4 % |
| | 156 | C₇H₇O₂S⁺ | 21,3 % |
| | 135 | C₄H₈Br⁺ | 40,2 % |

### b) Umsetzung des Produktgemisches aus a) mit 4-Hydroxybenzoesäuremethylester (Reaktand enthaltend die Gruppierung M)

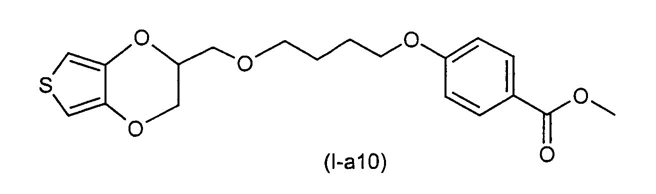

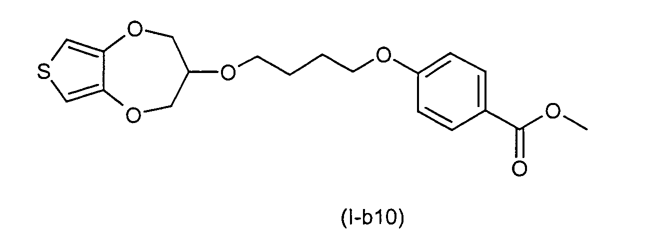

0,54 g (3,55 mmol) 4-Hydroxybenzoesäuremethylester, 1,23 g (4,00 mmol) des Produktgemisches aus a) (88 % EDT-Derivat, 12 % PDT-Derivat) und 3,39 g (10,4 mmol) Cs₂CO₃ werden in 30 ml DMF suspendiert und 4 d bei Raumtemperatur gerührt. Die Aufarbeitung erfolgt analog Beispiel 14 b).Das Rohprodukt wird säulenchromatographisch gereinigt (Laufmittel Hexan/Essigester 4 : 1). Man erhält ein gelbliches, viskoses Öl.
- **Ausbeute:**: 1,15 g (3,04 mmol = 85,6 % d. Theorie) (I-a10 mit einem Gehalt an PDT-Derivat von 16 %
**IR** ν⁻¹ [cm⁻¹] = 3111, 2948, 2872; 1711; 1604, 1578, 1483
^{**1**}**H-NMR** (500 MHz, CDCl₃): δ [ppm] = 7,97 (d, 2H, 14-H); 6,89 (d, 2H, 13-H); 6,47 (s, 16 % 2H, *A*-H); 6,32 (s, 84 % 2H, 1-H, 2-H); 4,39 - 4,26 (m, 84 % 1H, 6-H); 4,23 (dd, 84 % 1H, 5a-H überlagert dd von *Ca-*H); 4,12 (dd, 16 % 2H, *Cb*-H); 4,05 (dd, 84 % 1H, 5b-H überlagert von 11-H); 4,03 (t, 2H, 11-H überlagert von 5b-H); 3,88 (s, 3H, 17-H); 3,70 (dd, 84 % 1H, 7a-H); 3,62 (dd, 84 % 1H, 7b-H); 3,57 (t, 84 % 2H, 8-H); 1,98 - 1,84,1,84 - 1,69 (2*m, je 2H, 9-H, 10-H)
^{**13**}**C-NMR** (125,75 MHz, CDCl₃): δ [ppm] = 165,84; 161,73; 140,51, 140,48; 130,55; 141,41; 113,00; 98,68, 98,59; 71,60, 70,52; 68,16, 66,71, 65,11; 50,82; 25,11, 24,87

**Phasenübergänge** (bestimmt mit dem Polarisationsmikroskop):
K 40,8°C FC 48,7°C I (1. Heizen, < 1°C/min)

**Phasenübergänge** (bestimmt mit DSC):
*1. Heizen*: onset: 39,57°C, Maximum: 44,88°C, Endset: 50,06°C

| **Elementaranalyse:** | | |
|---|---|---|
| berechnet | 60,00 % C | 5,86 % H |
| gefunden | 60,20 % C | 6,01 % H |

| | | | |
|---|---|---|---|
| **MS** (EI, 70 eV): m/z = | 378 | M⁺ | 16,5 % |
| | 227 | C₁₁H₁₅O₃S⁺ | 26,0 % |
| | 155 | C₇H₇O₂S⁺ | 32,1 % |

### Beispiel 17: Kationisches, hochleitfähiges Polythiophen aus (I-a10) und (I-b10) als Beschichtung durch in situ-Polymerisation

0,165 g des gemäß Beispiel 16 hergestellten Thiophens (I-a10)/(I-b10) (Stoffmengen-Verhältnis 84 : 16) werden in 6,625 g n-Butanol warm gelöst. Zu der Lösung werden rasch 1,562 g einer 40 %igen Lösung von Eisen-III-tosylat in n-Butanol (Baytron® CB 40; Hersteller: H. C. Starck GmbH) gegeben und das Gemisch auf eine auf ca. 80°C vorgewärmte Glasplatte aufgerakelt (Nassfilm-Schichtdicke 24 µm). Nach 10 min Trocknung bei 100°C und Abkühlen auf Raumtemperatur (23°C) wird die Beschichtung mit entionisiertem Wasser gewaschen und föngetrocknet. Als Oberflächenwiderstand (Zweipunktmethode) werden 276 Ω/square gemessen. Die Platte wird nochmals 30 min bei 150°C getempert, als Oberflächenwiderstand nach Abkühlen auf 23°C werden 66 Ω/square gemessen.

### Beispiel 18:

### a) Umsetzung eines EDT-Methanol-Hydroxy-PDT Gemisches mit 1,5-Dibrompentan (Reaktand enthaltend die Gruppierung B):

Die Umsetzung entspricht Beispiel 12 a).

### b) Umsetzung des Produktgemisches aus a) mit 4'-Hydroxybiphenyl-4-carbonsäureethylester (Reaktand enthaltend die Gruppierung M)

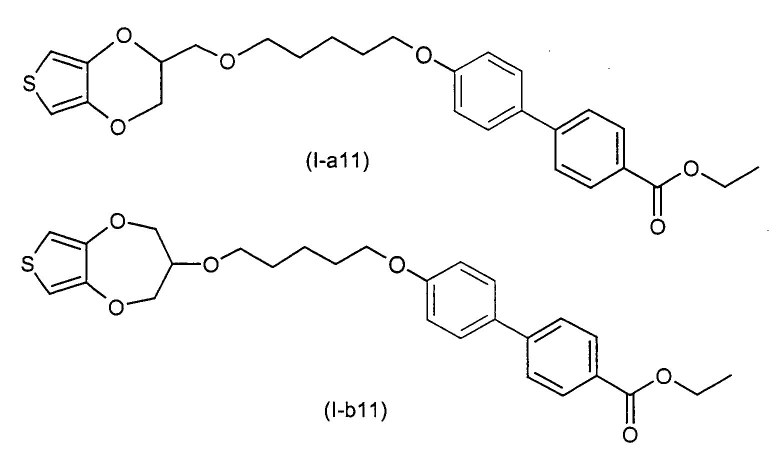

1,08 g (4,46 mmol) 4'-Hydroxybiphenyl-4-carbonsäureethylester, 1,50 g (4,67 mmol) des Produktgemisches aus a) (7 % PDT-Derivat, 93 % EDT-Derivat) und 1,55 g (4,76 mmol) Cs₂CO₃ werden in 40 ml DMF suspendiert und 4 d bei Raumtemperatur gerührt. Die Aufarbeitung erfolgt analog Beispiel 12 b).

Das Rohprodukt wird aus Methanol/Methylenchlorid umkristallisiert Der farblose Niederschlag wird abfiltriert, mit Methanol gewaschen und im Vakuum getrocknet.
- **Ausbeute:**: Fraktion 1: 1,08 g (2,24 mmol = 50,2 % d. Theorie) (I-a11) im Gemisch mit 3 % PDT-Derivat (I-b11)
Fraktion 2: 0,77 g (1,60 mmol = 35,8 % d. Theorie) (I-a11) im Gemisch mit 6 % PDT-Derivat (I-b11)
Fraktion 3: 0,18 g (0,37 mmol = 8,4 % d. Theorie) (I-a11) im Gemisch mit 30 % PDT-Derivat (I-b11)
**IR** ν⁻¹ [cm⁻¹] = 2946, 1899, 2869; 1703; 1600, 1581, 1489; 826
^{**1**}**H-NMR** (500 MHz, CDCl₃): δ [ppm] = 8,08 (d, 2H); 7,61 (d, 2H); 7,56 (d, 2H); 6,97 (d, 2H); 6,47 (s, 3 % 2H); 6,33, 6,32 (2*d, je 97 % 1H); 4,39 (q, 2H), 4,35 - 4,27 (m, 97 % 1H); 4,24 (dd, 1H,*J*_{CH-CH}: 11,66 Hz überlagert dd); 4,11 (dd, 3 % 2H); 4,06 (dd, 1H, *J*_{CH-CH}: 11,66 Hz); 4,01 (t, 2H); 3,69 (dd, 1H, *J*_{CH-CH}: 10,40 Hz); 3,61 (dd, 1H, *J*_{CH-CH}: 10,40 Hz); 3,54 (t, 2H); 1,87 - 1,79, 1,73 - 1,63, 1,59 - 1,51 (3*m, je 2H); 1,41 (t, 3H)
^{**13**}**C-NMR** (125,75 MHz, CDCl₃): δ [ppm] = 167,00; 159,68; 145,52; 141,97, 141,93; 132,70; 130,44, 128,73, 126,79; 128,94; 115,29; 100,09, 99,98; 73,04, 72,19; 69,58, 68,25, 66,61; 61,29; 29,68, 29,42; 23,08; 14,78

**Phasenübergänge** (bestimmt mit dem Polarisationsmikroskop):
K 87,7°C FC 91,6°C I (1. Heizen, < 1°C/min)
K 87,8°C FC 94,0°C I (2. Heizen, < 1°C/min) bei 70,8°C bereits FC, jedoch nach
   Tempern rekristallisiert

**Phasenübergänge** (bestimmt mit DSC)
*1. Heizen*: onset: 84,45°C, Maximum: 86,63°C, Endset: 90,08°C,
   Heizrate: 10°C/min

| **Elementaranalyse:** | | |
|---|---|---|
| berechnet | 67,20 % C | 6,27 % H |
| gefunden | 67,28 % C | 6,23 % H |

| | | | |
|---|---|---|---|
| **MS** (EI, 70 eV): m/z = | 482 | M⁺ | 68,5 % |
| | 241 | C₁₂H₁₇O₃S⁺ | 67,5 % |
| | 155 | C₇H₇O₂S⁺ | 40,6 % |

### Beispiel 19: Kationisches, hochleitfähiges Polythiophen aus (I-a11) und (I-b11) als Beschichtung durch in situ-Polymerisation

0,2111 g des gemäß Beispiel 18 hergestellten Thiophens (I-a11)/(I-b11) (Fraktion 1, Stoffmengen-Verhältnis 97 : 3) werden in 6,625 g n-Butanol gelöst. Zu der Lösung werden rasch 1,562 g einer 40 %igen Lösung von Eisen-III-tosylat in n-Butanol (Baytron® CB 40; Hersteller: H. C. Starck GmbH) gegeben und das Gemisch auf eine auf ca. 80°C vorgewärmte Glasplatte aufgerakelt (Nassfilm-Schichtdicke 24 µm). Nach 10 min Trocknung bei 80°C und Abkühlen auf Raumtemperatur (23°C) wird die Beschichtung mit entionisiertem Wasser gewaschen und föngetrocknet. Als Oberflächenwiderstand (Zweipunktmethode) werden 170 Ω/square gemessen. Die Platte wurde nochmals 30 min bei 150° getempert, als Oberflächenwiderstand nach Abkühlen auf 23°C werden 58 Ω/square gemessen.

## Patentansprüche

1. 3,4-Alkylendioxythiophene **dadurch gekennzeichnet, dass** sie gegebenenfalls über ein Bindeglied B mit einer mesogenen Gruppe substituiert sind,
ausgenommen das 3,4-Alkylendioxythiophen der Formel (i)

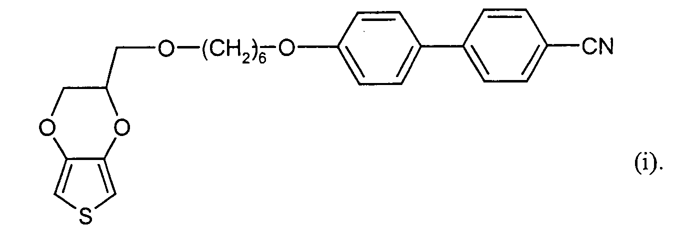

2. 3,4-Alkylendioxythiophene gemäß Anspruch 1, **dadurch gekennzeichnet, dass** es Verbindungen der allgemeinen Formel (I) sind, 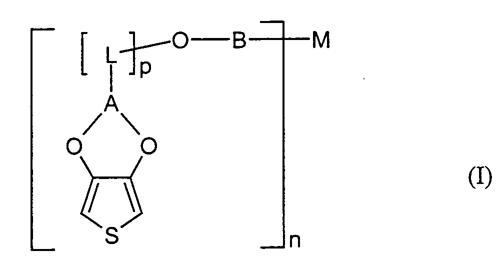 worin
A für einen C₁-C₅-Alkylenrest steht, der an beliebiger Stelle durch einen Linker L substituiert ist und gegebenenfalls weitere Substituenten trägt,
L für eine Methylengruppe steht,
p für 0 oder eine ganze Zahl von 1 bis 6 steht,
M für eine n-funktionelle mesogene Gruppe steht,
n für eine ganze Zahl von 1 bis 8 steht und
B für ein Bindeglied der allgemeinen Formel (B) steht 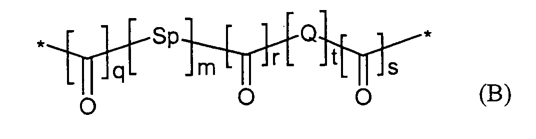
worin
q für 0 oder 1 steht,
r, s für 0 oder 1 stehen, mit der Maßgabe, dass wenn r für 1 steht s für 0 steht und umgekehrt oder beide für 0 stehen können,
t für 0 oder 1 steht,
Sp für einen Spacer ausgewählt aus gegebenenfalls substituierten linearen oder cylischen C₁-C₂₀-Alkylen-, C₅-C₂₀-Arylen-, C₂-C₂₀-Heteroarylen-, wobei zusätzlich noch ein bis drei Heteroatome ausgewählt aus N, O oder S im heteroaromatischen Ring- oder Ringsystem vorhanden sein können, C₆-C₂₀-Aralkylen-, C₂-C₂₀₀-Oligo- oder -Polyethergruppen steht,
m für 0 oder 1 steht,
Q für O, S oder NH steht.

3. 3,4-Alkylendioxythiophene gemäß Anspruch 1 oder 2, **dadurch gekennzeichnet, dass**
M für eine n-funktionelle Gruppe der allgemeinen Formeln (II-a) oder (II-b) steht, 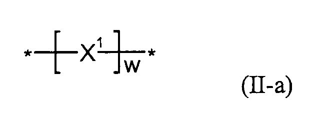 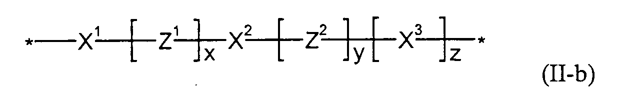
worin
X¹,X²,X³ unabhängig voneinander für gegebenenfalls substituierte Strukturen ausgewählt aus 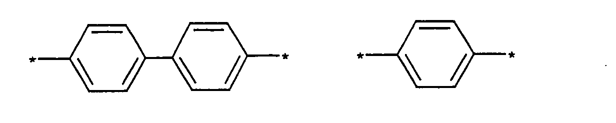 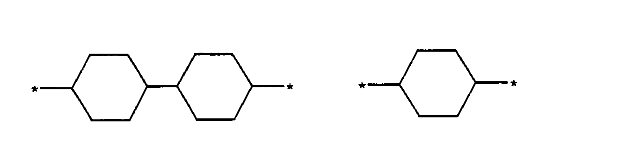 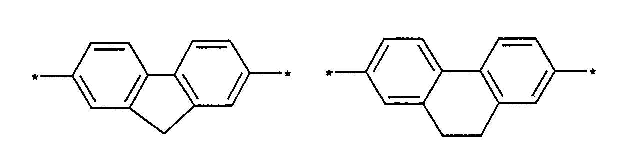 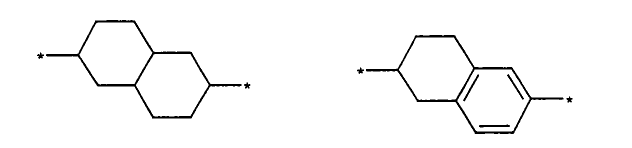 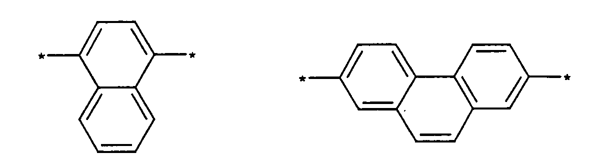 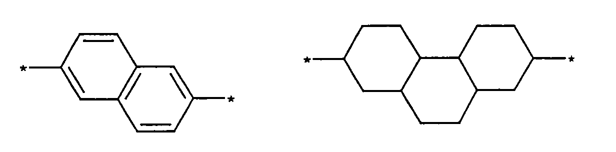 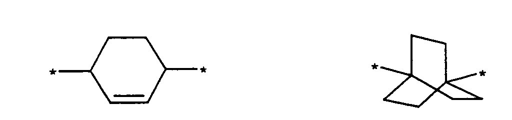 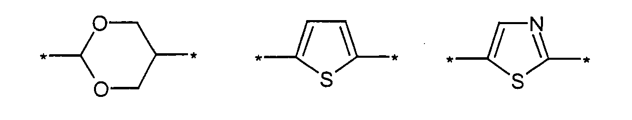 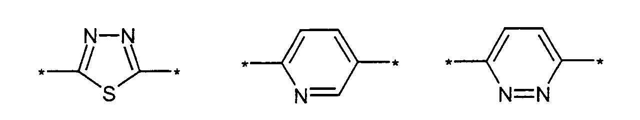 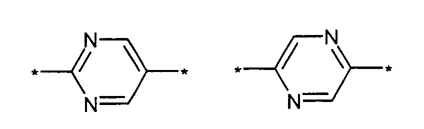 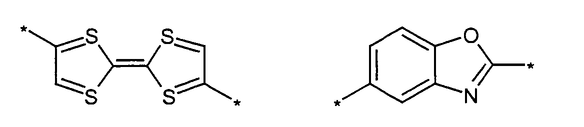 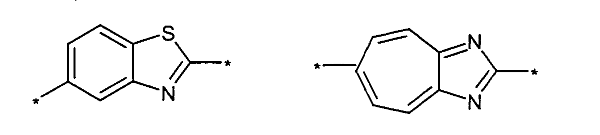 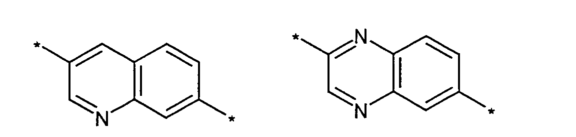 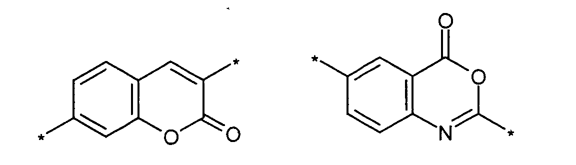 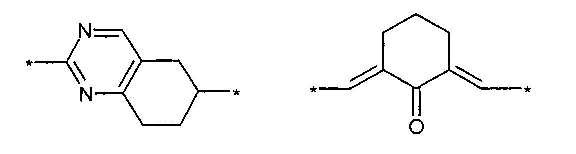 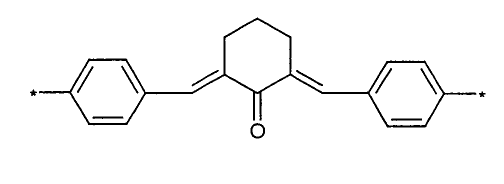
Z¹, Z² unabhängig voneinander für Strukturen ausgewählt aus 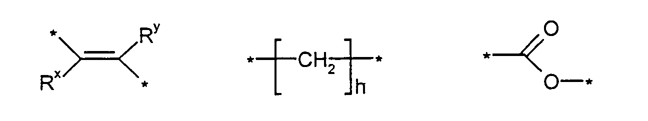 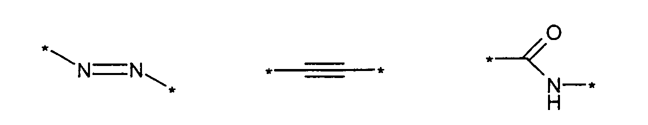 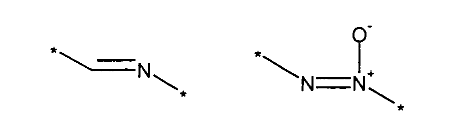 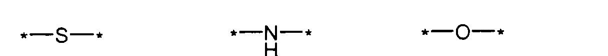 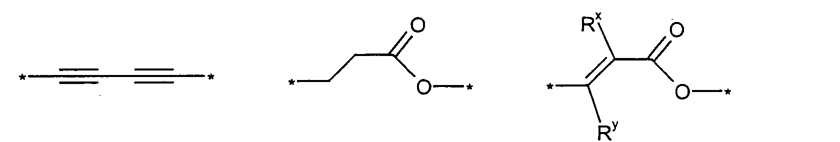 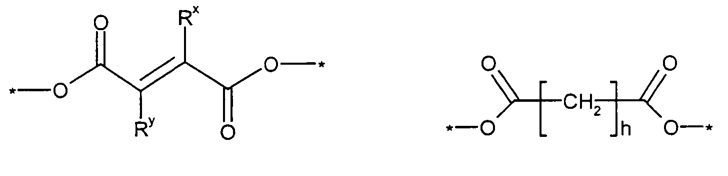
steht,
wobei
R^{x} und R^{y} unabhängig voneinander für H, gegebenenfalls substituiertes C₁-C₂₂-Alkyl, C₁-C₂₂-Halogenalkyl, C₁-C₂₂-Alkenyl, C₁-C₂₂-Alkoxy, C₁-C₂₂-Thioalkyl, C₁-C₂₂-Iminoalkyl, C₁-C₂₂-Alkoxycarbonyl, C₁-C₂₂-Alkoxycarbonyloxy, einen Rest einer aliphatischen C₁-C₂₂-Alkancarbonsäure oder der Acrylsäure, Halogen, Pseudohalogen, NO₂ eine Carboxyl- oder eine Hydroxygruppe steht,
h für eine ganze Zahl von 1 bis 10 steht,
w eine ganze Zahl von 1 bis 5 ist,
x, y, z unabhängig voneinander für 0 oder 1 stehen, und
n für 1 oder 2 steht, wobei
für den Fall, dass n für 1 steht, die Gruppe der allgemeinen Formeln (II-a) oder (II-b) an einer der mit * gekennzeichneten Verknüpfungsstellen eine Flügelgruppe F trägt,
wobei
F für H, gegebenenfalls substituiertes C₁-C₂₂-Alkyl, C₁-C₂₂-Halogenalkyl, C₁-C₂₂-Alkenyl, C₁-C₂₂-Alkoxy, C₁-C₂₂-Thioalkyl, C₁-C₂₂-Iminoalkyl, C₁-C₂₂-Alkoxycarbonyl, C₁-C₂₂-Alkoxycarbonyloxy, einen Rest einer aliphatischen C₁-C₂₂-Alkancarbonsäure oder der Acrylsäure, Halogen, Pseudohalogen, eine Nitro- (NO₂-), eine Carboxyl-, eine Sulfonsäure- bzw. Sulfonat- oder eine Hydroxygruppe steht.

4. 3,4-Alkylendioxythiophene gemäß Anspruch 1 oder 2, **dadurch gekennzeichnet, dass**
M für eine n-funktionelle Gruppe ausgewählt aus den allgemeinen Formeln (II-c-1) bis (II-c-6) steht, 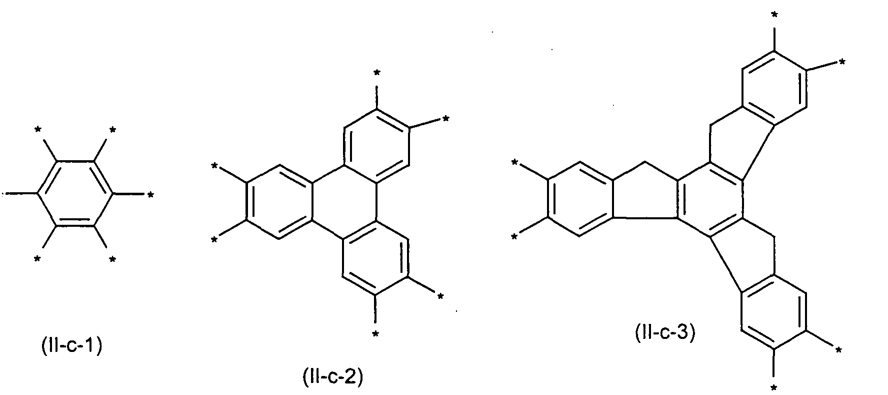 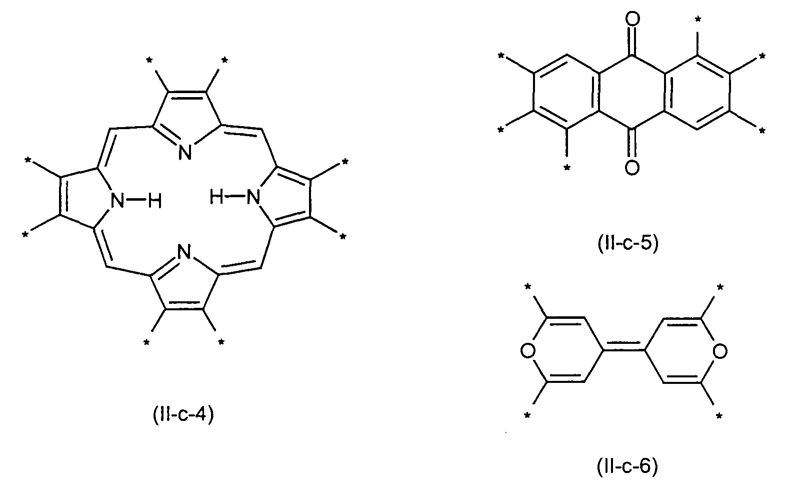
worin
n für eine ganze Zahl von 1 bis 8 steht, wobei
für den Fall, dass n für eine ganze Zahl kleiner als 8 steht, die Gruppe ausgewählt aus den allgemeinen Formeln (II-c-1) bis (II-c-6) an den restlichen 8-n mit * gekennzeichneten Verknüpfungsstellen eine Flügelgruppe F trägt,
wobei
F für H, gegebenenfalls substituiertes C₁-C₂₂-Alkyl, C₁-C₂₂-Halogenalkyl, C₁-C₂₂-Alkenyl, C₁-C₂₂-Alkoxy, C₁-C₂₂-Thioalkyl, C₁-C₂₂-Iminoalkyl, C₁-C₂₂-Alkoxycarbonyl, C₁-C₂₂-Alkoxycarbonyloxy, einen Rest einer aliphatischen C₁-C₂₂-Alkancarbonsäure oder der Acrylsäure, Halogen, Pseudohalogen, eine Nitro- (NO₂-), eine Carboxyl-, eine Sulfonsäure- bzw. Sulfonat- oder eine Hydroxygruppe steht.

5. 3,4-Alkylendioxythiophene gemäß Anspruch 1 oder 2, **dadurch gekennzeichnet, dass**
M für einen Steroidrest oder ein Derivat eines Steroidrestes steht.

6. 3,4-Alkylendioxythiophene gemäß Anspruch 5, **dadurch gekennzeichnet, dass**
M für einen Cholesterylrest oder ein Derivat des Cholesterylrestes der allgemeinen Formel (III-a) steht, 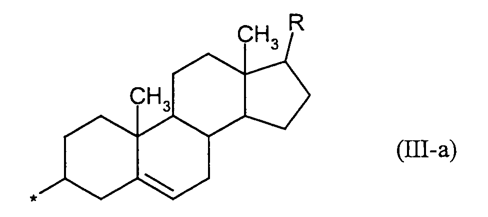
worin
R für H, gegebenenfalls substituiertes Ci-C22-Alkyl, C₁-C₂₂-Halogenalkyl, C₁-C₂₂-Alkenyl, C₁-C₂₂-Alkoxy, C₁-C₂₂-Thioalkyl, C₁-C₂₂-Iminoalkyl, C₁-C₂₂-Alkoxycarbonyl, C₁-C₂₂-Alkoxycarbonyloxy, einen Rest einer aliphatischen C₁-C₂₂-Alkancarbonsäure oder der Acrylsäure, Halogen, Pseudohalogen, eine Nitro- (NO₂-), eine Carboxyl-, eine Sulfonsäure- bzw. Sulfonat- oder eine Hydroxygruppe steht.

7. 3,4-Alkylendioxythiophene oder Mischungen aus 3,4-Alkylendioxythiophenen gemäß wenigstens einem der Ansprüche 1 bis 6, **dadurch gekennzeichnet, dass** sie eine Struktur der allgemeinen Formel (I-a) und/oder (I-b) besitzen, 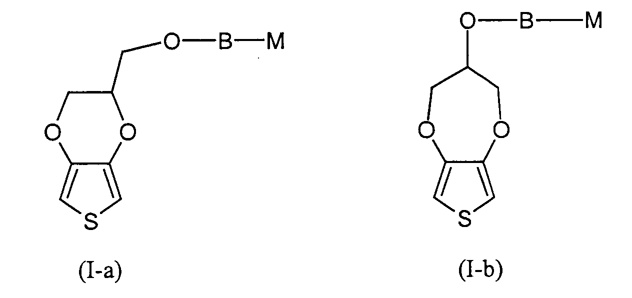 worin
B und M die in wenigstens einem der Ansprüche 2 bis 6 genannte Bedeutung haben.

8. Verwendung der 3,4-Alkylendioxythiophene oder Mischungen aus 3,4-Alkylendioxythiophenen gemäß wenigstens einem der Ansprüche 1 bis 7 zur Herstellung von Polythiophenen.

9. Polythiophene, **dadurch gekennzeichnet, dass** sie wiederkehrende Einheiten der allgemeinen Formel (IV) enthalten, 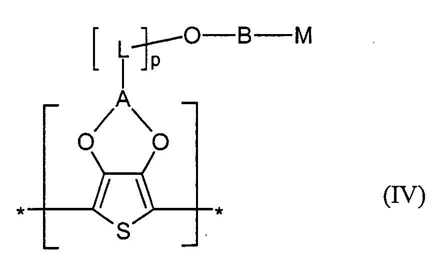 worin
A, L, p, M und B die in wenigstens einem der Ansprüche 2 bis 6 genannte Bedeutung haben,
ausgenommen Polythiophene bestehend aus wiederkehrenden Einheiten der Formel (ii)

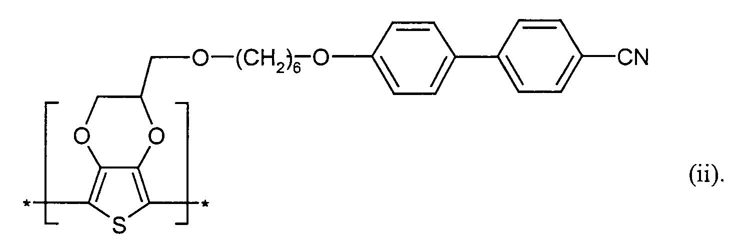

10. Polythiophene gemäß Anspruch 9, **dadurch gekennzeichnet, dass** sie wiederkehrende Einheiten der allgemeinen Formel (IV-a) und/oder (IV-b) enthalten, 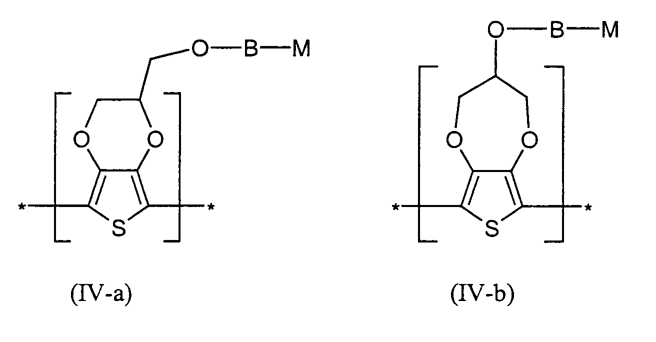 worin
M und B die in wenigstens einem der Ansprüche 2 bis 6 genannte Bedeutung haben.

11. Polythiophene gemäß Anspruch 9 oder 10, **dadurch gekennzeichnet, dass** sie kationisch und elektrisch leitfähig sind und zur Kompensation der positiven Ladung Anionen als Gegenionen gebunden enthalten.

12. Polythiophene gemäß Anspruch 11, **dadurch gekennzeichnet, dass** die Gegenionen Polyanionen von polymeren Carbonsäuren oder polymeren Sulfonsäuren sind.

13. Polythiophene gemäß Anspruch 9 oder 10, **dadurch gekennzeichnet, dass** sie neutral und halbleitend sind.

14. Verfahren zur Herstellung von Polythiophenen gemäß wenigstens einem der Ansprüche 9 bis 13, **dadurch gekennzeichnet, dass** Verbindungen der allgemeinen Formel (I), 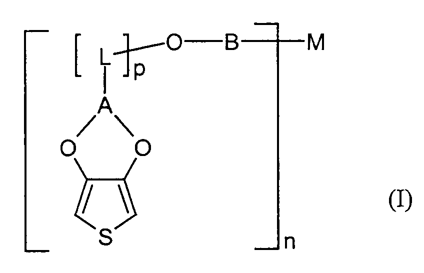 worin
A, L, p, M und B die in wenigstens einem der Ansprüche 2 bis 6 genannte Bedeutung haben,
elektrochemisch oxidativ polymerisiert werden.

15. Verfahren zur Herstellung von Polythiophenen, **dadurch gekennzeichnet, dass** Verbindungen der allgemeinen Formel (I), 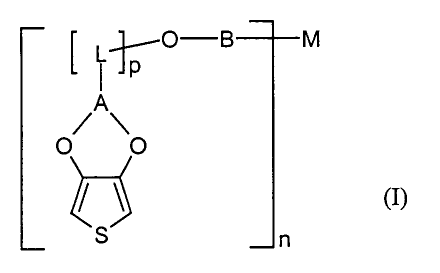 worin
A für einen C₁-C₅-Alkylenrest steht, der an beliebiger Stelle durch einen Linker L substituiert ist und gegebenenfalls weitere Substituenten trägt,
L für eine Methylengruppe steht,
p für 0 oder eine ganze Zahl von 1 bis 6 steht,
n für eine ganze Zahl von 1 bis 8 steht und
B für ein Bindeglied der allgemeinen Formel (B) steht 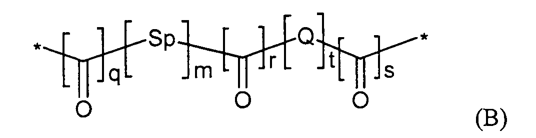
worin
q für 0 oder 1 steht,
r, s für 0 oder 1 stehen, mit der Maßgabe, dass wenn r für 1 steht s für 0 steht und umgekehrt oder beide für 0 stehen können,
t für 0 oder 1 steht,
Sp für einen Spacer ausgewählt aus gegebenenfalls substituierten linearen oder cylischen C₁-C₂₀-Alkylen-, C₅-C₂₀-Arylen-, C₂-C₂₀-Heteroarylen-, wobei zusätzlich noch ein bis drei Heteroatome ausgewählt aus N, O oder S im heteroaromatischen Ring- oder Ringsystem vorhanden sein können, C₆-C₂₀-Aralkylen-, C₂-C₂₀₀-Oligo- oder -Polyethergruppen steht,
m für 0 oder 1 steht,
Q für O, S oder NH steht, und
M die in wenigstens einem der Ansprüche 2 bis 6 genannte Bedeutung hat,
chemisch oxidativ polymerisiert werden.

16. Polythiophene **dadurch gekennzeichnet, dass** sie wiederkehrende Einheiten der allgemeinen Formel (IV) enthalten, 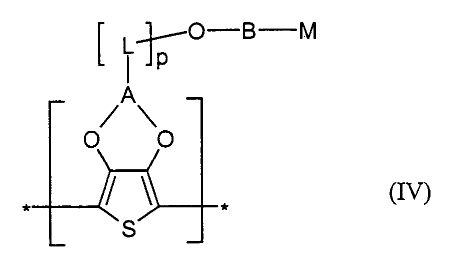 worin
A, L, p und B die in Anspruch 15 genannte Bedeutung haben und
M die in wenigstens einem der Ansprüche 2 bis 6 genannte Bedeutung hat,
erhältlich durch ein Verfahren gemäß Anspruch 15.

17. Verwendung der Polythiophene gemäß wenigstens einem der Ansprüche 9 bis 13 und 16 als Bestandteil in elektrischen oder elektronischen Bauteilen, lichtemittierenden Bauteilen, zur antistatischen Beschichtung, in der Optoelektronik oder in der Solarenergie-Technik.

18. Verwendung der Polythiophene gemäß wenigstens einem der Ansprüche 9 bis 13 und 16 zur Herstellung leitfähiger Schichten.
